# FASCICULE DE BREVET EUROPEEN

(11) **EP 1 341 506 B1**
(45) Date de publication et mention de la délivrance du brevet: **03.10.2007**
(21) Numéro de dépôt: 01999221.3
(22) Date de dépôt: 22.11.2001
(51) Int. Cl.: A61K 8/73, A61K 8/81, A61K 8/87, A61K 8/88, A61Q 5/10

(54) **COMPOSITION DE TEINTURE POUR FIBRES KERATINIQUES COMPRENANT UN POLYMERE ASSOCIATIF ET UN POLYMERE A MOTIFS ACRYLAMIDE, HALOGENURE DE DIALKYLDIALLYLAMMONIUM, ET ACIDE CARBOXYLIQUE VINYLIQUE**
FÄRBEZUSAMMENSETZUNG FÜR KERATINÖSE FASERN MIT EINEM ASSOZIATIVEN POLYMER UND EINEM POLYMER MIT ACRYLAMIDEINHEITEN, DIALKYLDIALLYLAMMONIUMHALID UND VINYLCARBONSÄURE
DYEING COMPOSITION FOR KERATINOUS FIBRES COMPRISING AN ASSOCIATIVE POLYMER AND A POLYMER WITH ACRYLAMIDE UNITS, DIALKYLDIALLYLAMMONIUM HALIDE, AND VINYLIC CARBOXYLIC ACID

(30) Priorité: 04.12.2000 FR 0015682
(43) Date de publication de la demande: 10.09.2003
(73) Titulaire: L'ORÉAL, 75008 Paris (FR)
(72) Inventeur: COTTARD, François, F-92400 Courbevoie (FR); RONDEAU, Christine, F-78500 Sartrouville (FR)
(74) Mandataire: Wattremez, Catherine
(86) Numéro de dépôt international: PCT/FR2001/003693
(87) Numéro de publication internationale: WO 2002/045674

(56) Documents cités:
- WO-A-94/10968
- WO-A-97/44002
- WO-A-99/13822
- WO-A-99/36047
- WO-A-99/37278
- DE-A- 19 905 615
- FR-A- 2 794 364
- US-A- 5 876 463
- US-A- 5 976 517

## Description

L'invention concerne une composition pour la teinture et notamment la teinture d'oxydation des fibres kératiniques, en particulier des fibres kératiniques humaines et plus particulièrement les cheveux, comprenant, dans un milieu approprié pour la teinture, au moins un colorant d'oxydation et/ou un colorant direct et au moins un polymère associatif et qui est caractérisée par le fait qu'elle comprend en outre au moins un polymère à motifs (i) acrylamide, (ii)halogénure de dialkyldiallylammonium, et (iii) acide carboxylique vinylique.
L'invention concerne également les procédés et dispositifs de teinture mettant en oeuvre ladite composition de teinture.

Dans le domaine capillaire, on peut distinguer deux types de coloration.

Le premier est la coloration semi-permanente ou temporaire, ou coloration directe, qui fait appel à des colorants capables d'apporter à la coloration naturelle des cheveux, une modification de couleur plus ou moins marquée résistant éventuellement à plusieurs shampooings. Ces colorants sont appelés colorants directs; ils peuvent être mis en oeuvre avec ou sans agent oxydant. En présence d'oxydant, le but est d'obtenir une coloration directe éclaircissante. La coloration éclaircissante est mise en oeuvre en appliquant sur les cheveux le mélange extemporané d'un colorant direct et d'un oxydant et permet notamment d'obtenir, par éclaircissement de la mélanine des cheveux, un effet avantageux tel qu'une couleur unie dans le cas des cheveux gris ou de faire ressortir la couleur dans le cas de cheveux naturellement pigmentés.

Le deuxième est la coloration permanente ou coloration d'oxydation. Celle-ci est réalisée avec des colorants dits "d'oxydation" comprenant les précurseurs de coloration d'oxydation et les coupleurs. Les précurseurs de coloration d'oxydation, appelés couramment "bases d'oxydation", sont des composés initialement incolores ou faiblement colorés qui développent leur pouvoir tinctorial au sein du cheveu en présence d'agents oxydants ajoutés au moment de l'emploi, en conduisant à la formation de composés colorés et colorants. La formation de ces composés colorés et colorants résulte, soit d'une condensation oxydative des "bases d'oxydation" sur elles-mêmes, soit d'une condensation oxydative des "bases d'oxydation" sur des composés modificateurs de coloration appelés couramment "coupleurs" et généralement présents dans les compositions tinctoriales utilisées en teinture d'oxydation.
La variété des molécules mises en jeu, qui sont constituées d'une part, par les bases d'oxydation et d'autre part, par les coupleurs, permet l'obtention d'une palette très riche en coloris.
Pour varier encore les nuances obtenues avec lesdits colorants d'oxydation, ou les enrichir de reflets, Il arrive qu'on leur ajoute des colorants directs.

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Pour localiser le produit de coloration d'oxydation à l'application sur les cheveux afin qu'il ne coule pas sur le visage ou en dehors des zones que l'on se propose de teindre, outre l'emploi d'épaississants traditionnels tels que l'acide polyacrylique réticulé, les hydroxyéthylcelluloses, les cires, et des mélanges d'agents tensio-actifs non-ioniques de HLB (Hydrophilic Lipophilic Balance), convenablement choisis, on a aussi eu recours à des polymères associatifs de type anionique, non-ionique ou cationique.
Les polymères associatifs de type anionique, non-ionique ou cationique représentent un progrès important dans la recherche d'une solution à ce problème.

Voici maintenant que la demanderesse a découvert, de façon tout à fait inattendue et surprenante, de nouvelles compositions de teinture, optimales quant à l'application sur les fibres (ne coulent pas et restent donc mieux localisées au point d'application), quant à obtenir des nuances puissantes et chromatiques avec de faibles sélectivités et de bonnes ténacités, quant aux propriétés cosmétiques conférées à la chevelure traitée, lesdites compositions de teinture comprenant dans la composition colorante, ou dans la composition oxydante (lorsqu'il s'agit de teinture d'oxydation ou de teinture directe éclaircissante), ou dans les deux compositions à la fois, outre au moins un polymère associatif non ionique, cationique ou amphotère une quantité efficace d'au moins un polymère à motifs (i)acrylamide, (ii)halogénure de dialkyldiallylammonium, et (iii) acide carboxylique vinylique.
Cette découverte est à la base de la présente invention.

La présente invention a ainsi pour objet une nouvelle composition pour la teinture des fibres kératiniques, en particulier des fibres kératiniques humaines, telles que les cheveux, comprenant, dans un milieu approprié pour la teinture, au moins un colorant et au moins un polymère associatif non ionique, cationique ou amphotère et qui est caractérisée par le fait qu'elle contient en outre au moins un polymère à motifs (i) acrylamide, (ii)halogénure de dialkyldiallylammonium, et (iii) acide carboxylique vinylique.
Selon l'invention, ledit colorant peut être un colorant direct ou un colorant d'oxydation (base d'oxydation et/ou coupleur).

Un autre objet de la présente invention porte sur une composition prête à l'emploi pour la teinture des fibres kératiniques, en particulier des fibres kératiniques humaines, telles que les cheveux comprenant au moins un colorant (direct ou d'oxydation), au moins un polymère associatif non ionique, cationique ou amphotère au moins un polymère à motifs (i) acrylamide, (ii)halogénure de dialkyldiallylammonium, et (iii) acide carboxylique vinylique, et en outre au moins un agent oxydant.

Au sens de la présente invention, on entend par composition prête à l'emploi, toute composition destinée à être appliquée immédiatement sur les fibres kératiniques; elle peut donc être stockée telle quelle avant utilisation ou résulter du mélange extemporané de deux ou plusieurs compositions.

L'invention vise également un procédé de teinture des fibres kératiniques, et en particulier des fibres kératiniques humaines telles que les cheveux, consistant à appliquer sur les fibres au moins une composition colorante comprenant, dans un milieu approprié pour la teinture, au moins un colorant (direct ou d'oxydation), la couleur étant révélée à pH alcalin, neutre ou acide, à l'aide d'une composition oxydante contenant au moins un agent oxydant qui est mélangée juste au moment de l'emploi à la composition colorante ou qui est appliquée séquentiellement sans rinçage intermédiaire, au moins un polymère associatif non ionique, cationique ou amphotère et au moins un polymère à motifs (i) acrylamide, (ii)halogénure de dialkyldiallylammonium, et (iii) acide carboxylique vinylique étant présents dans la composition colorante ou dans la composition oxydante ou dans chacune desdites compositions.

Dans un procédé préféré, les polymères associatif(s) et les polymères à motifs (i) acrylamide, (ii)halogénure de dialkyldiallylammonium, et (iii) acide carboxylique vinylique sont présents dans la composition colorante.

L'invention a également pour objet des dispositifs de teinture ou « kits » à plusieurs compartiments.

Un dispositif à 2 compartiments selon l'invention comprend un compartiment renfermant une composition colorante comprenant, dans un milieu approprié pour la teinture, au moins un colorant (direct ou d'oxydation), et un autre compartiment renfermant une composition oxydante comprenant, dans un milieu approprié pour la teinture, un agent oxydant, au moins un polymère associatif et au moins un polymère à motifs (i) acrylamide, (ii)halogénure de dialkyldiallylammonium, et (iii) acide carboxylique vinylique étant présents dans la composition colorante ou la composition oxydante, ou dans chacune de ces compositions.

Mais d'autres caractéristiques, aspects, objets et avantages de l'invention apparaîtront encore plus clairement à la lecture de la description et des exemples qui suivent.

### Polymères à motifs (i) acrylamide, (ii)halogénure de dialkyldiallylammonium, et (iii) acide carboxylique vinylique, utilisés selon l'invention.

Selon l'invention, on entend désigner par motifs acrylamide des motifs de structure (I): 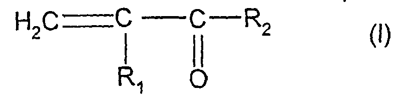 dans laquelle,
- R₁ désigne H ou CH₃,
- R₂ est choisi parmi un radical amino, diméthylamino, tert-butylamino, dodécylamino, ou -NH-CH₂OH.
Le motif acrylamide [formule (I) dans laquelle R₁ est H et R₂ est amino] est particulièrement préféré.

Les motifs halogénure de dialkyldiallylammonium sont de structure (II) suivante: 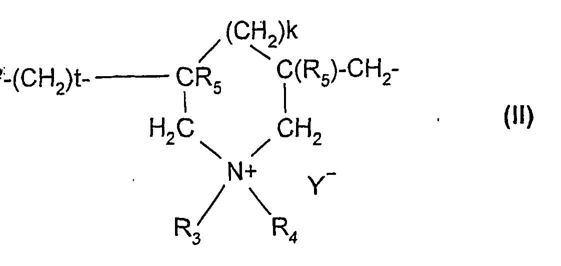 formule dans laquelle k et t sont égaux à 0 ou 1, la somme k + t étant égale à 1 ; R₅ désigne un atome d'hydrogène ou un radical méthyle ; R₃ et R₄, indépendamment l'un de l'autre, désignent un groupement alkyle ayant de 1 à 4 atomes de carbone, un groupement hydroxyalkyle dans lequel le groupement alkyle a de préférence 1 à 5 atomes de carbone, un groupement amidoalkyle(C₁-C₄), ou R₃ et R₄ peuvent désigner conjointement avec l'atome d'azote auquel ils sont rattachés, des groupements hétérocycliques, tels que pipéridinyle ou morpholinyle ; R₃ et R₄ indépendamment l'un de l'autre désignent de préférence un groupement alkyle ayant de 1 à 4 atomes de carbone ; Y est un anion tel que bromure, chlorure, acétate, borate, citrate, tartrate, bisulfate, bisulfite, sulfate, phosphate.
Parmi ces motifs halogénure de dialkyldiallylammonium, on préfère le chlorure de diméthyldiallylammonium.

Selon l'invention, on entend également désigner par motifs acide carboxylique vinylique, l'acide acrylique, l'acide méthacrylique et l'acide 2-acrylamido-2-méthyl-propane sulfonique.
Le motif acide acrylique est particulièrement préféré.

Parmi ces polymères à motifs (i) acrylamide, (ii)halogénure de dialkyldiallylammonium, et (iii) acide carboxylique vinylique, on peut citer notamment les terpolymères acrylamide / chlorure de diméthyldiallylammonium / acide acrylique, répertoriés dans le dictionnaire C.T.F.A. International Cosmetic Ingredient Dictionary, 7ème édition 1997, sous la dénomination "POLYQUATERNIUM 39" ; des exemples de tels polymères ont les compositions pondérales suivantes :
- (25/50/25), proposé par la société CALGON,
   sous l'appellation MERQUAT PLUS 3330 DRY®,
   en solution aqueuse à 10% sous l'appellation MERQUAT PLUS 3330®,
   en solution aqueuse à 23,6% sous l'appellation POLYMER 3413-54,
   en solution aqueuse à 23,3% sous l'appellation POLYMER 3413-58,
   en solution aqueuse à 21,3% sous l'appellation POLYMER 3413-62,
- (50/40/10), proposé par la société CALGON,
   en solution aqueuse à 5% sous l'appellation POLYMER 3120-59,
- (50/35/15), proposé par la société CALGON,
   en solution aqueuse à 5% sous l'appellation POLYMER 3120-61,
- (50/30/20), proposé par la société CALGON,
   en solution aqueuse à 4,6% sous l'appellation POLYMER 3120-63;
on peut également citer le terpolymère acrylamide / chlorure de diméthyldiallylammonium / acide 2-acrylamido-2-méthyl-propane sulfonique proposé en solution aqueuse à 8% par la société CALGON sous l'appellation POLYMER 3575-53.

Selon l'invention, le ou les polymères à motifs (i)acrylamide, (ii)halogénure de dialkyldiallylammonium, et (iii)acide carboxylique vinylique représentent environ 0,1% à 10% en poids, de préférence environ 0,5% à 5% en poids, et plus particulièrement environ 1 % à 3% en poids du poids total de la composition de teinture.

### Polymères associatifs utilisés selon l'invention

Les polymères associatifs sont des polymères hydrosolubles, capables dans un milieu aqueux, de s'associer réversiblement entre eux ou avec d'autres molécules.
Leur structure chimique comprend des zones hydrophiles, et des zones hydrophobes caractérisées par au moins une chaîne grasse.
Les polymères associatifs selon l'invention peuvent être de type cationique, amphotère et de préférence non ionique.

### Polymères associatifs de type cationique

On peut citer parmi eux :
-(I) les polyuréthanes associatifs cationiques dont la famille a été décrite par la demanderesse dans la demande de brevet français N°-0009609; elle peut être représentée par la formule générale (la) suivante :

R-X-(P)ₙ-[L-(Y)ₘ]ᵣ-L'-(P')ₚ-X'-R' (Ia)

dans laquelle:
R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate ;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000 ; la molécule contenant au moins une fonction amine protonée ou quaternisée et au moins un groupement hydrophobe.

Dans un mode de réalisation préféré de ces s polyuréthanes, les seuls groupements hydrophobes sont les groupes R et R' aux extrémités de chaîne.

Une famille préférée de polyuréthanes associatifs cationiques est celle correspondant à la formule (la) décrite ci-dessus et dans laquelle :
R et R' représentent tous les deux indépendamment un groupement hydrophobe,
X, X' représentent chacun un groupe L",
n et p valent entre 1 et 1000 et
L, L', L", P, P', Y et m ont la signification indiquée ci-dessus.

Une autre famille préférée de polyuréthanes associatifs cationiques est celle correspondant à la formule (la) ci-dessus dans laquelle :
R et R' représentent tous les deux indépendamment un groupement hydrophobe, X,
X' représentent chacun un groupe L", n et p valent 0, et L, L', L", Y et m ont la signification indiquée ci-dessus.
Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Encore une autre famille préférée de polyuréthanes associatifs cationiques est celle correspondant à la formule (la) ci-dessus dans laquelle :
R et R' représentent tous les deux indépendamment un groupement hydrophobe,
X et X' représentent tous les deux indépendamment un groupement comportant une amine quaternaire,
n et p valent zéro, et
L, L', Y et m ont la signification indiquée ci-dessus.

La masse moléculaire moyenne en nombre des polyuréthanes associatifs cationiques est comprise de préférence entre 400 et 500 000, en particulier entre 1000 et 400 000 et idéalement entre 1000 et 300 000.
Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.
Préférentiellement, le groupement hydrocarboné provient d'un composé monofonctionnel.
A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Lorsque X et/ou X' désignent un groupement comportant une amine tertiaire ou quaternaire, X et/ou X' peuvent représenter l'une des formules suivantes: 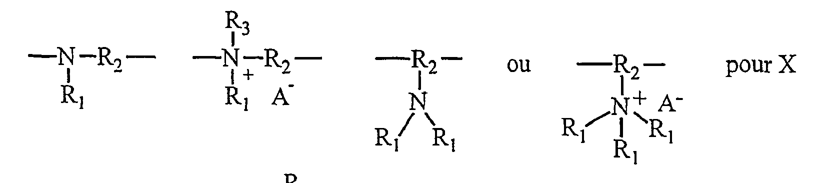 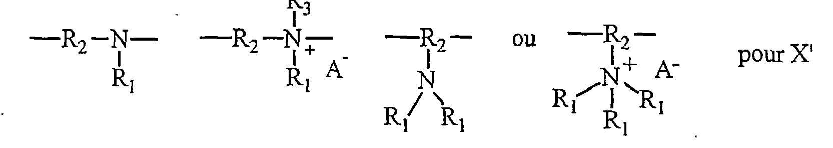 dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O; P ;
R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
A-est un contre-ion physiologiquement acceptable.

Les groupements L, L' et L" représentent un groupe de formule: 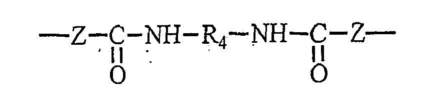 dans laquelle :
Z représente -O-, -S- ou -NH- ; et
R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

Les groupements P et P', comprenant une fonction amine peuvent représenter au moins l'une des formules suivantes : 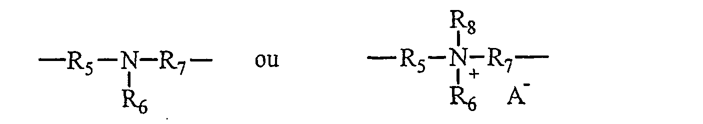 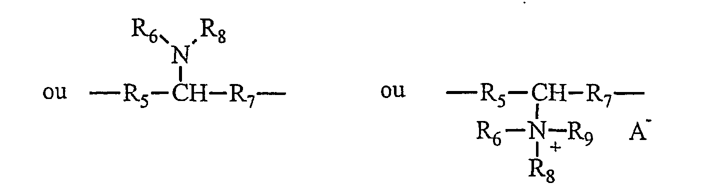 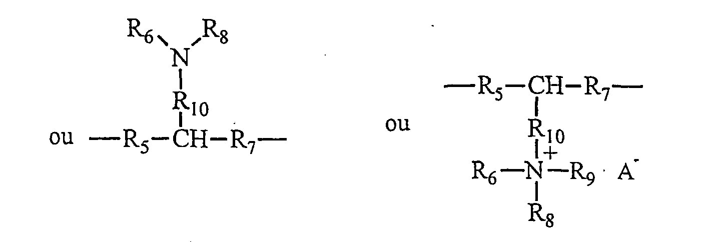 dans lesquelles :
R₅ et R₇ ont les mêmes significations que R₂ défini précédemment;
R₆, R₈ et R₉ ont les mêmes significations que R₁ et R₃ définis précédemment ;
R₁₀ représente un groupe alkylène, linéaire ou ramifié, éventuellement insaturé et pouvant contenir un ou plusieurs hétéroatomes choisis parmi N, O, S et P, et A⁻ est un contre-ion physiologiquement acceptable.

En ce qui concerne la signification de Y, on entend par groupement hydrophile, un groupement hydrosoluble polymérique ou non.

A titre d'exemple, on peut citer, lorsqu'il ne s'agit pas de polymères, l'éthylèneglycol, le diéthylèneglycol et le propylèneglycol.
Lorsqu'il s'agit, conformément à un mode de réalisation préféré, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthanes associatifs cationiques de formule (la) selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" de la présente invention englobe ces trois types de polymères à savoir les polyuréthanes proprement dits, les polyurées et les polythiourées ainsi que des copolymères de ceux-ci.

Un premier type de composés entrant dans la préparation du polyuréthane de formule (la) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.
Comme indiqué précédemment, ce composé peut comporter plus d'un motif à fonction amine. Il s'agit alors d'un polymère portant une répétition du motif à fonction amine. Ce type de composés peut être représenté par l'une des formules suivantes :

HZ-(P)ₙ-ZH,

ou

HZ-(P')ₚ-ZH

dans lesquelles Z, P, P', n et p sont tels que définis plus haut.

A titre d'exemple de composé à fonction amine, on peut citer la N-méthyldiéthanolamine, la N-tert-butyl-diéthanolamine, la N-sulfoéthyldiéthanolamine.

Le deuxième composé entrant dans la préparation du polyuréthane de formule (la) est un diisocyanate correspondant à la formule :

O=C=N-R₄-N=C=O

dans laquelle R₄ est défini plus haut.
A titre d'exemple, on peut citer le méthylènediphényl-diisocyanate, le méthylènecyclohexanediisocyanate, l'isophorone-diisocyanate, le toluénediisocyanate, le naphtalènediisocyanate, le butanediisocyanate, l'hexanediisocyanate.

Un troisième composé entrant dans la préparation du polyuréthane de formule (la) est un composé hydrophobe destiné à former les groupes hydrophobes terminaux du polymère de formule (la).
Ce composé est constitué d'un groupe hydrophobe et d'une fonction à hydrogène labile, par exemple une fonction hydroxyle, amine primaire ou secondaire, ou thiol.
A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné - hydroxyle.
Le groupe hydrophobe du polyuréthane de formule (la) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.

Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire, ou thiol. Ce composé peut être un polymère terminé aux extrémités des chaînes par l'une de ces fonctions à hydrogène labile.
A titre d'exemple, on peut citer, lorsqu'il ne s'agit pas de polymères, l'éthylèneglycol, le diéthylèneglycol et le propylèneglycol.

Lorsqu'il s'agit d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Le groupe hydrophile noté Y dans la formule (la) est facultatif. En effet, les motifs à fonction amine quaternaire ou protonée peuvent suffire à apporter la solubilité ou l'hydrodispersibilité nécessaire pour ce type de polymère dans une solution aqueuse.
Bien que la présence d'un groupe Y hydrophile soit facultative, on préfère cependant des polyuréthanes associatifs cationiques comportant un tel groupe.
-(II) les dérivés de cellulose quaternisée et les polyacrylates à groupements latéraux aminés non cycliques.
Les dérivés de cellulose quaternisée sont en particulier,
- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.
Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.
On peut indiquer comme exemples d'alkylhydroxyéthyl-celluloses quaternisées à chaînes grasses en C₈-C₃₀, les produits QUATRISOFT LM 200®, QUATRISOFT LM-X 529-18-A®, QUATRISOFT LM-X 529-18B® (alkyle en C₁₂) et QUATRISOFT LM-X 529-8® (alkyle en C₁₈) commercialisés par la société AMERCHOL et les produits CRODACEL QM®, CRODACEL QL® (alkyle en C₁₂) et CRODACEL QS® (alkyle en C18) commercialisés par la société CRODA.

### Polymères associatifs amphotères

Ils sont choisis de préférence parmi ceux comportant au moins un motif cationique non cyclique. Plus particulièrement encore, on préfère ceux préparés à partir ou comprenant 1 à 20 moles% de monomère comportant une chaîne grasse, et de préférence 1,5 à 15 moles% et plus particulièrement encore 1,5 à 6 moles%, par rapport au nombre total de moles de monomères.

Les polymères associatifs amphotères préférés selon l'invention comprennent, ou sont préparés en copolymérisant :
1) au moins un monomère de formule (Ia) ou (Ib): 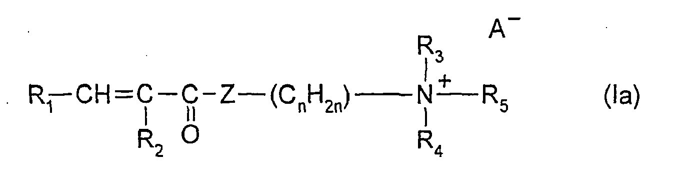 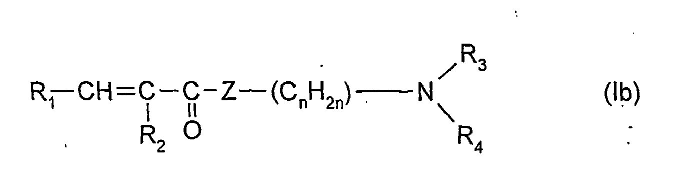 dans lesquelles, R₁ et R₂, identiques ou différents, représentent un atome d'hydrogène ou un radical méthyle, R₃, R₄ et R₅, identiques ou différents, représente un radical alkyle linéaire ou ramifié ayant de 1 à 30 atomes de carbone,
   Z représente un groupe NH ou un atome d'oxygène,
   n est un nombre entier de 2 à 5,
   A- est un anion issu d'un acide organique ou minéral, tel qu'un anion méthosulfate ou un halogénure tel que chlorure ou bromure;
2) au moins un monomère de formule (II)

   R₆-CH = CR₇ - COOH (II)

   dans laquelle, R₆ et R₇, identiques ou différents, représentent un atome d'hydrogène ou un radical méthyle;
   et
3) au moins un monomère de formule (III):

   R₆-CH= CR₇- COXR₈ (III)
dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone ;
l'un au moins des monomères de formule (Ia), (Ib) ou (III) comportant au moins une chaîne grasse.

Les monomères de formule (Ia) et (Ib) de la présente invention sont choisis, de préférence, dans le groupe constitué par:
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide,
ces monomères étant éventuellement quaternisés, par exemple par un halogénure d'alkyle en C₁-C₄ ou un sulfate de dialkyle en C₁-C₄.

Plus particulièrement, le monomère de formule (la) est choisi parmi le chlorure d'acrylamidopropyl triméthyl ammonium et le chlorure de méthacrylamidopropyl triméthyl ammonium.

Les monomères de formule (II) de la présente invention sont choisis, de préférence, dans le groupe constitué par l'acide acrylique, l'acide méthacrylique, l'acide crotonique et l'acide méthyl-2 crotonique. Plus particulièrement, le monomère de formule (II) est l'acide acrylique.

Les monomères de formule (III) de la présente invention sont choisis, de préférence, dans le groupe constitué par des acrylates ou méthacrylates d'alkyle en C₁₂-C₂₂ et plus particulièrement en C₁₆-C₁₈.

Les monomères constituant les polymères amphotères à chaîne grasse de l'invention sont de préférence déjà neutralisés et/ou quaternisés.

Le rapport du nombre de charges cationiques/charges anioniques est de préférence égal à environ 1.

Les polymères associatifs amphotères selon l'invention comprennent de préférence de 1 à 10 moles% du monomère comportant une chaîne grasse (monomère de formule (la), (Ib) ou (III)), et de préférence de 1,5 à 6 moles %.

Les poids moléculaires moyens en poids des polymères associatifs amphotères selon l'invention peuvent varier de 500 à 50.000.000 et sont de préférence compris entre 10.000 et 5 000 000.

Les polymères associatifs amphotères selon l'invention peuvent également contenir d'autre monomères tels que des monomères non ioniques et en particulier tels que les acrylates ou méthacrylates d'alkyle en C₁-C₄.

Des polymères associatifs amphotères selon l'invention sont par exemple décrits et préparés dans la demande de brevet WO9844012.
Parmi les polymères associatifs amphotères selon l'invention, on préfère les terpolymères acide acrylique/chlorure de (méth)acrylamidopropyl triméthyl ammonium/ méthacrylate de stéaryle.

Dans la composition de teinture selon l'invention, on préfère utiliser un polymère associatif de type non ionique.

### Polymères associatifs de type non ionique

Selon l'invention, ils sont choisis de préférence parmi :
- **(1)** les celluloses modifiées par des groupements comportant au moins une chaîne grasse;
   on peut citer à titre d'exemple:
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS® (alkyles en C₁₆) vendu par la société AQUALON, ou le produit BERMOCOLL EHM 100® vendu par la société BEROL NOBEL,
   - celles modifiées par des groupes polyalkylène glycol éther d'alkyl phénol, tel que le produit AMERCELL POLYMER HM-1500® (polyéthylène glycol (15) éther de nonyl phénol) vendu par la société AMERCHOL.
- **(2)** les hydroxypropylguars modifiés par des groupements comportant au moins une chaîne grasse tel que le produit ESAFLOR HM 22® (chaîne alkyle en C₂₂) vendu par la société LAMBERTI, les produits RE210-18® (chaîne alkyle en C₁₄) et RE205-1® (chaîne alkyle en C₂₀) vendus par la société RHONE POULENC.
- **(3)** les copolymères de vinyl pyrrolidone et dé monomères hydrophobes à chaîne grasse dont on peut citer à titre d'exemple :
   - les produits ANTARON V216® ou GANEX V216® (copolymère vinylpyrrolidone / hexadécène) vendu par la société I.S.P..
   - les produits ANTARON V220® ou GANEX V220® (copolymère vinylpyrrolidone eicosène) vendu par la société I.S.P.
- **(4)** les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208®.
- **(5)** les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
- **(6)** les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
- **(7)** les polymères à squelette aminoplaste éther possédant au moins une chaîne grasse, tels que les composés PURE THIX® proposés par la société SUD-CHEMIE.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.
Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.
Par extension figurent aussi parmi les polyéthers polyuréthanes non-ioniques à chaîne grasse, ceux dont les séquences hydrophiles sont liées aux séquences lipophiles par d'autres liaisons chimiques.
A titre d'exemples de polyéthers polyuréthanes non-ioniques à chaîne grasse utilisables dans l'invention, on peut aussi utiliser aussi le Rhéolate 205® à fonction urée vendu par la société RHEOX ou encore les Rhéolates® 208 , 204 ou 212, ainsi que l'Acrysol RM 184®.
On peut également citer le produit ELFACOS T210® à chaîne alkyle en C₁₂₋₁₄ et le produit ELFACOS T212® à chaîne alkyle en C₁₈ de chez AKZO.
Le produit DW 1206B® de chez ROHM & HAAS à chaîne alkyle en C₂₀ et à liaison uréthanne, proposé à 20 % en matière sèche dans l'eau, peut aussi être utilisé.
On peut aussi utiliser des solutions ou dispersions de ces polymères notamment dans l'eau ou en milieu hydroalcoolique. A titre d'exemple, de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.
Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Plus particulièrement encore, selon l'invention, on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.
De tels polyéther polyuréthanes sont vendus notamment par la société ROHM & HAAS sous les appellations Aculyn 46® et Aculyn 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange, de propylèneglycol (39%) et d'eau (26%)].

Les polymères associatifs de type non ionique, anionique, cationique ou amphotère sont utilisés de préférence en une quantité pouvant varier d'environ 0,1 à 10% en poids du poids total de la composition de teinture. Plus préférentiellement, cette quantité varie d'environ 0,5 à 5% en poids, et encore plus particulièrement d'environ 1 à 3% en poids.

### Colorants d'oxydation

Les colorants d'oxydation utilisables selon l'invention sont choisis parmi les bases d'oxydation et/ou les coupleurs.
De préférence les compositions selon l'invention contiennent au moins une base d'oxydation.

La nature de ces bases d'oxydation n'est pas critique. Elles peuvent notamment être choisies parmi les ortho- et para-phénylènediamines, les bases doubles, les ortho- et para- aminophénols, les bases hétérocycliques suivantes ainsi que les sels d'addition de tous ces composés avec un acide.

On peut notamment citer :
- **(I)** les paraphénylènediamines de formule (I) suivante et leurs sels d'addition avec un acide : 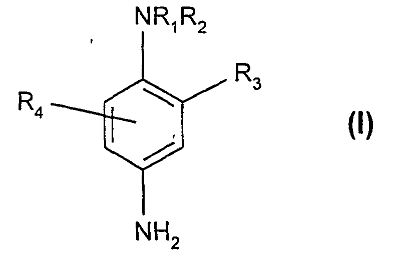 dans laquelle :
   R₁ représente un atome d'hydrogène, un radical alkyle en C₁-C₄, monohydroxyalkyle en C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle ;
   R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
   R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
   R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
   R₄ représente un atome d'hydrogène, d'halogène ou un radical alkyle en C₁-C₄.

   Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les paraphénylènediamines de formule (I) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.
   Parmi les paraphénylènediamines de formule (I) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.
- **(II)** Selon l'invention, on entend par bases doubles, les composés comportant au moins deux noyaux aromatiques sur lesquels sont portés des groupements amino et/ou hydroxyle.
   Parmi les bases doubles utilisables à titre de bases d'oxydation dans les compositions tinctoriales conformes à l'invention, on peut notamment citer les composés répondant à la formule (II) suivante, et leurs sels d'addition avec un acide : 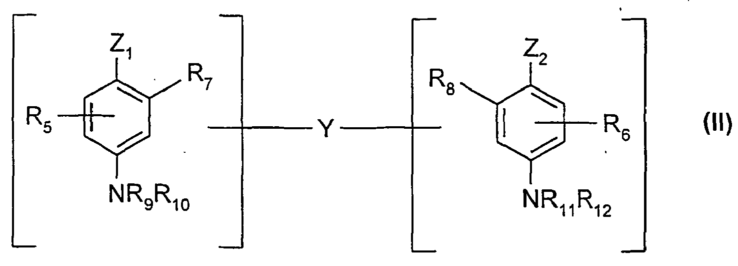 dans laquelle:
   - Z₁ et Z₂, identiques ou différents, représentent un radical hydroxyle ou -NH₂ pouvant être substitué par un radical alkyle en C₁-C₄ ou par un bras de liaison Y ;
   - le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆;
   - R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
   - R₇, R₈, R₉, R₁₀, R₁₁ et R₁₂, identiques ou différents, représentent un atome d'hydrogène, un bras de liaison Y ou un radical alkyle en C₁-C₄ ;
   étant entendu que les composés de formule (II) ne comportent qu'un seul bras de liaison Y par molécule.
   Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les bases doubles de formules (II) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.
   Parmi ces bases doubles de formule (II), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.
- (III) les para-aminophénols répondant à la formule (III) suivante, et leurs sels d'addition avec un acide : 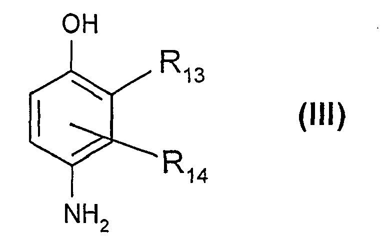 dans laquelle :
   R₁₃ représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
   R₁₄ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

   Parmi les para-aminophénols de formule (III) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.
   - **(IV)** les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.
- **(V)** parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diarrine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyi-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

Selon la présente invention, les bases d'oxydation représentent de préférence de 0,0005 à 12% en poids environ du poids total de la composition et encore plus préférentiellement de 0,005 à 8% en poids environ de ce poids.

La composition tinctoriale conforme à l'invention peut renfermer un ou plusieurs coupleurs choisis parmi ceux classiquement utilisés en teinture d'oxydation et notamment parmi les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α,-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Généralement le ou les coupleurs représentent de préférence de 0,0001 à 15% en poids environ du poids total de la composition tinctoriale et encore plus préférentiellement de 0,001 à 10% environ.

Les sels d'addition avec un acide de ces colorants d'oxydation (bases et/ou coupleurs) sont notamment choisis parmi les chlorhydrates, les bromhydrates, les sulfates et les tartrates, les lactates et les acétates.

La composition tinctoriale conforme à l'invention comprenant au moins un colorant d'oxydation peut en outre renfermer un ou plusieurs colorants directs notamment pour modifier les nuances obtenues avec les bases et/ou les coupleurs en les enrichissant de reflets. Ces colorants directs peuvent notamment être choisis parmi les colorants nitrés, azoïques ou anthraquinoniques, neutres, cationiques ou anioniques, classiquement utilisés ou ceux décrits notamment dans les demandes de brevet FR-2782450, 2782451, 2782452 et EP-1025834, dans la proportion pondérale d'environ 0,001 à 20% et de préférence de 0,01 à 10% du poids total de la composition.

### Colorants directs

Les colorants directs convenant pour la teinture directe sont ceux décrits ci-dessus et ceux convenant plus particulièrement pour la teinture éclaircissante (c'est à dire avec un oxydant) sont ceux décrits notamment dans les demandes de brevet FR-2782450, 2782451, 2782452 et EP-1025834.

### Milieu

Le milieu de la composition approprié pour la teinture, est de préférence un milieu aqueux constitué par de l'eau pouvant comprendre avantageusement des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations comprises entre environ 0,5 et 20% et, de préférence, entre environ 2 et 10% en poids par rapport au poids total de la composition.

La composition tinctoriale et/ou la composition oxydante (dans le cas d'une teinture d'oxydation ou d'une teinture éclaircissante) peuvent en outre plus particulièrement comprendre, au moins un agent tensio-actif anionique, non ionique, cationique ou amphotère ou zwittérionique dans la proportion d'au moins 0,01% en poids par rapport au poids total de la composition, et de préférence un agent tensio-actif de nature non-ionique.

### Ces agents tensio-actifs peuvent être choisis parmi :

### Les tensioactifs non ioniques :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀- C₁₄) amines ou les oxydes de N-acylaminopropylmorpholine.

### Les tensioactifs anioniques :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### Les tensioactifs amphotères ou zwittérioniques:

Les agents tensioactifs amphotères ou zwitterioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.
Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :
R₂ -CONHCH₂CH₂ -N(R₃)(R₄)(CH₂COO⁻)
dans laquelle: R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C)

dans laquelle :
B représente -CH₂CH₂OX', C représente -(CH₂)_{Z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂' désigne un radical alkyle d'un acide Rg -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Coco-amphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL^{®} C2M concentré par la société RHODIA CHIMIE.

### Les tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition de teinture selon l'invention peuvent varier de 0,01 à 40% et de préférence de 0,5 à 30% du poids total de la composition.

De préférence, selon l'invention, la composition colorante et/ou la composition oxydante peuvent en outre plus particulièrement comprendre, au moins un polymère cationique ou amphotère (différent des polymères associatifs et des polymères à motifs (i) acrylamide, (ii)halogénure de dialkyldiallylammonium, et (iii) acide acrylique, selon l'invention) dans la proportion d'au moins 0,01% en poids par rapport au poids total de la composition.
Plus particulièrement selon l'invention, les dits polymères cationiques ou amphotères sont dans la partie colorante.

### Polymères cationiques

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.
Ce sont des produits connus. Ils sont notamment décrits dans les brevets français n° 2 505 348 ou 2 542 997. Parmi lesdits polymères, on peut citer :
**(1)** Les homopolymères ou copolymères dérivés d'esters ou d'amides acryliques ou méthacryliques et comportant au moins un des motifs de formules (I), (II), (III) ou (IV) suivantes: 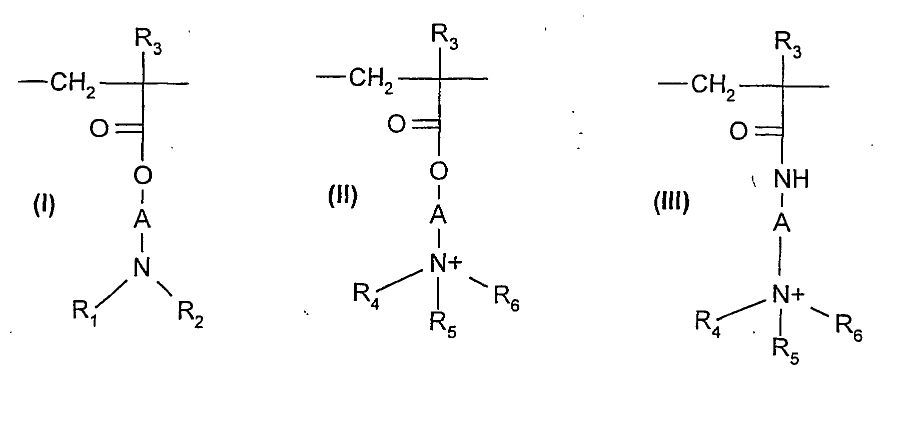 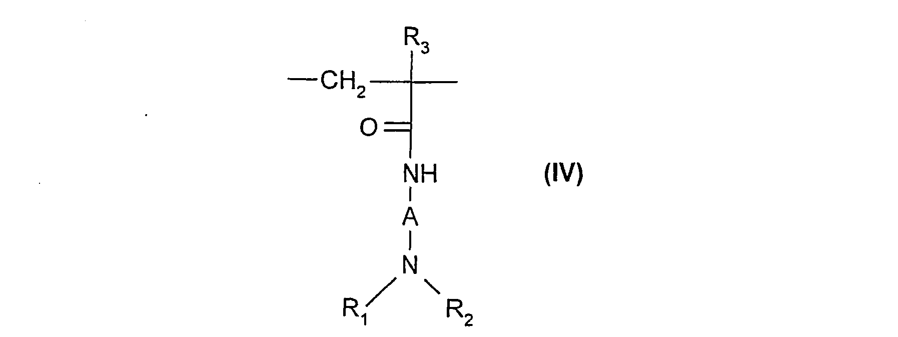 dans lesquelles:
   R₃ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate
   ou un halogénure tel que chlorure ou bromure.
   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques. Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle, tel que celui vendu sous la dénomination HERCOFLOC® par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT P 100® par la société CIBA GEIGY,
   - le copolymère d'acrylamide et de méthosulfate de méthacryloyloxyéthyltriméthylammonium vendu sous la dénomination RETEN® par la société HERCULES,
   - les copolymères vinylpyrrolidone / acrylate ou méthacrylate de dialkylaminoalkyle quaternisés ou non, tels que les produits vendus sous la dénomination "GAFQUAT®" par la société ISP comme par exemple "GAFQUAT 734" ou "GAFQUAT 755" ou bien les produits dénommés "COPOLYMER 845®, 958® et 937®". Ces polymères sont décrits en détail dans les brevets français 2.077.143 et 2.393.573,
   - les terpolymères méthacrylate de diméthyl amino éthyle/ vinylcaprolactamel vinylpyrrolidone tel que le produit vendu sous la dénomination GAFFIX VC 713® par la société ISP,
   - les copolymère vinylpyrrolidone / méthacrylamidopropyl dimethylamine commercialisés notamment sous la dénomination STYLEZE CC 10® par ISP,
   - et les copolymères vinylpyrrolidone / méthacrylamide de diméthylaminopropyle quaternisés tel que le produit vendu sous la dénomination "GAFQUAT HS 100®" par la société ISP.
**(2)** Les dérivés d'éthers de cellulose comportant des groupements ammonium quaternaire décrits dans le brevet français 1 492 597, et en particulier les polymères commercialisés sous les dénominations "JR®" (JR 400, JR 125, JR 30M) ou "LR®" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
**(3)** Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200®" et "Celquat H 100®" par la Société National Starch.
**(4)** Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S®, JAGUAR C 15®, JAGUAR C 17® ou JAGUAR C162® par la société MEYHALL.
**(5)** Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
**(6)** Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure, d'alkyle ou encore par un oligomère résultant de la réaction d'un composé bifonctionnel réactif vis-à-vis d'une bis-halohydrine, d'un bis-azétidinium, d'une bis-haloacyldiamnne, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.
**(7)** Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine® F, F4 ou F8" par la société Sandoz.
**(8)** Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57®" par la société Hercules Inc. ou bien sous la dénomination de "PD 170®"
   ou "Delsette 101®" par la société Hercules dans le cas du copolymère d'acide adipiquelépoxypropylidiéthylène-triamine.
**(9)** Les cyclopolymères de dialkyl diallyl ammonium tels que les homopolymères ou copolymères comportant comme constituant principal de la chaîne des motifs répondant à la formule (V) : 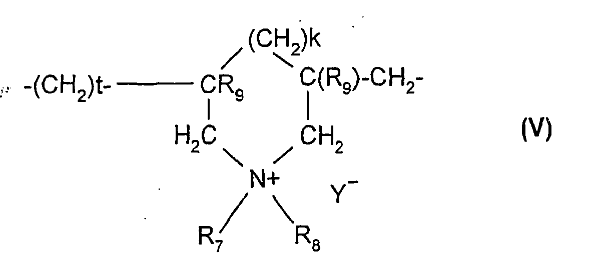 dans laquelle k et t sont égaux à 0 ou 1, la somme k + t étant égale à 1;
   R₇ et R₈, identiques ou différents, désignent un groupement alkyle ayant de 1 à 8 atomes de carbone, un groupement hydroxyalkyle(C₁-C₅), un groupement amidoalkyle(C₁-C₄), ou R₇ et R₈ désignent conjointement avec l'atome d'azote auquel ils sont rattachés, un groupement pipéridinyle ou morpholinyle ;
   R₉ désigne un atome d'hydrogène ou un radical méthyle ;
   R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100®" par la société Calgon (et ses homologues de faible masse moléculaire moyenne en poids) et les copolymères de chlorure de diallyldiméthylammonium et d'acrylamide commercialisés sous la dénomination "MERQUAT 550®".
**(10)** Le polymère de diammonium quaternaire contenant des motifs récurrents répondant à la formule : 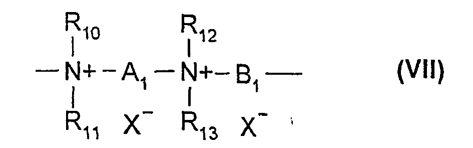 formule (VII) dans laquelle:
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R_{11,} R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrite, ester, acyle, amide ou -CO-O- R₁₄-D ou -CO-NH-R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement - (CH₂)n-CO-D-OC-(CH₂)n- dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH-.

   De préférence, X- est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (VIII) suivante: 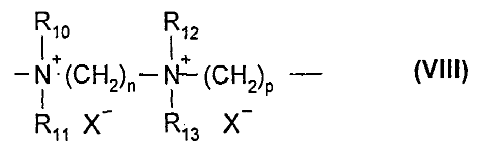 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.
**(11)** Les polymères de polyammonium quaternaire constitués de motifs récurrents de formule (IX) : 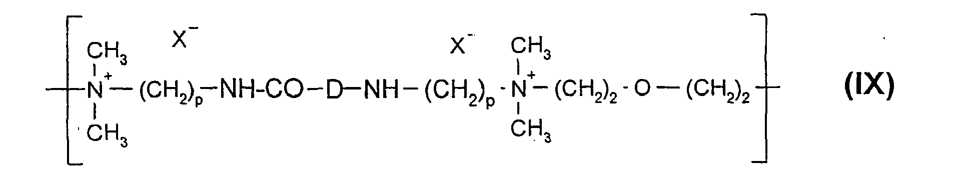 dans laquelle p désigne un nombre entier variant de 1 à 6 environ, D peut être nul ou peut représenter un groupement -(CH₂)ᵣ -CO- dans lequel r désigne un nombre égal à 4 ou à 7, X- est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
   Parmi eux, on peut par exemple citer, les produits "Mirapol A 15®", "Mirapol AD1®", "Mirapol AZ1®" et "Mirapol 175®" vendus par la société Miranol.
**(12)** Les polymères quaternaires de vinylpyrrolidone et de vinylimidazole tels que par exemple les produits commercialisés sous les dénominations Luviquat FC 905®, FC 550® et FC 370® par la société B.A.S.F.
**(13)** Les polyamines comme le Polyquart H® vendu par HENKEL, référencé sous le nom de " POLYETHYLENEGLYCOL (15) TALLOW POLYAMINE " dans le dictionnaire CTFA.
**(14)** Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE^{®} SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE^{®} SC 95 " et " SALCARE^{®} SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (9), (10) et (11) et encore plus préférentiellement les suivants :
-1/ l'homopolymère de chlorure de diméthyldiallylammonium (famille 9);
-2/ les polymères aux motifs récurrents de formule (VIII) décrite ci-avant (famille 10) et pour laquelle
R₁₀, R₁₁, R₁₂ et R₁₃ représentent un radical méthyle, n = 3, p = 6 et X = Cl, et notamment ceux dont le poids moléculaire, déterminé par chromatographie par perméation de gel, est compris entre 9500 et 9900 [Polymère W de formule ci-dessous]; R₁₀ et R₁₁ représentent un radical méthyle, R₁₂ et R₁₃ représentent un radical éthyle et n = p = 3 et X = Br, et notamment ceux dont le poids moléculaire, déterminé par chromatographie par perméation de gel, est d'environ 1200 [Polymère U de formule ci-dessous].

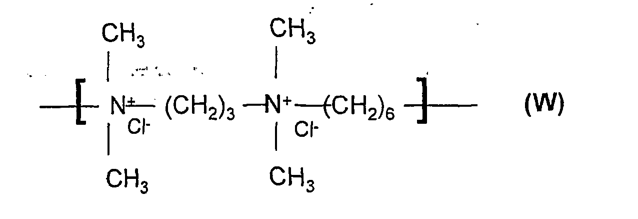

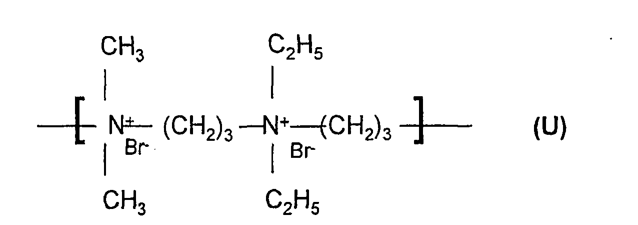

Lesdits polymères à motifs (W) et (U) sont préparés et décrits dans le brevet français 2 270 846.
-3/ les polymères à motifs de formule (IX) décrite ci-dessus (famille 11), pour laquelle, p est égal à 3, et,
a) D désigne la valeur zéro, X désigne un atome de chlore, la masse moléculaire mesurée par RMN du Carbone 13, (RMN¹³C) étant d'environ 25500 ; un polymère de ce type est vendu par la société MIRANOL sous le nom MIRAPOL-A15®,
b) D représente un groupement -(CH₂)₄-CO-, X désigne un atome de chlore, la masse moléculaire mesurée par RMN du Carbone 13 (RMN¹³C) étant d'environ 5600; un polymère de ce type est proposé par la société MIRANOL sous le nom de MIRAPOL-AD1®,
c) D représente un groupement -(CH₂)₇-CO-, X désigne un atome de chlore, la masse moléculaire mesurée par RMN du Carbone 13 (RMN¹³C) étant d'environ 8100 ; un polymère de ce type est proposé par la société MIRANOL sous le nom de MIRAPOL-AZ1®,
d) un " Block Copolymer " formé de motifs correspondant aux polymères décrits aux alinéas a) et b), proposé par la société MIRANOL sous les noms MIRAPOL-9®, (masse moléculaire RMN¹³C, environ 7800) MIRAPOL-175®, (masse moléculaire RMN¹³C, environ 8000) MIRAPOL-95®, (masse moléculaire RMN¹³C, environ 12500). Plus particulièrement encore, on préfère selon l'invention, le polymère à motifs de formule (IX) dans laquelle p est égal à 3, D désigne la valeur zéro, X désigne un atome de chlore, la masse moléculaire mesurée par RMN du Carbone 13 (RMN¹³C) étant d'environ 25500 (Mirapol-A15®).

### Polymères amphotères

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033® par la Société HENKEL.
   Le composé vinylique peut être également un sel de dialkyldiallylammonium tel que le chlorure de diméthyldiallylammonium. Les copolymères d'acide acrylique et de ce dernier monomère sont proposés sous les appelations MERQUAT 280® et MERQUAT 295®, par la société CALGON.
(2) Les polymères comportant des motifs dérivant:
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
      Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
      Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
      Les comonomères basiques préférés sont des méthacrylates d'aminoéthyle, de butyl aminoéthyle, de N,N'-diméthylaminoéthyle, de N-tertio-butylaminoéthyle.
      On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER® ou LOVOCRYL 47® par la société NATIONAL STARCH.
(3) Les polyaminoamides réticulés et alcoylés partiellement ou totalement dérivant de polyaminoamides de formule générale :

   +CO-R₁₉-CO-Z+ (X)

   dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 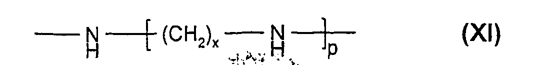 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (XI) ci-dessus, dans lequel x=2 et p=1 et qui dérive de l'éthylènediamine, ou le radical dérivant de la pipérazine : 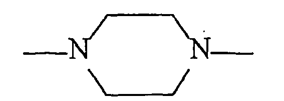
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
      Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique. Les alcanes sultones utilisées dans l'alcoylation sont de préférence la propane ou la butane sultone, les sels des agents d'alcoylation sont de préférence les sels de sodium
   ou de potassium.
(4) Les polymères comportant des motifs zwittérioniques de formule : 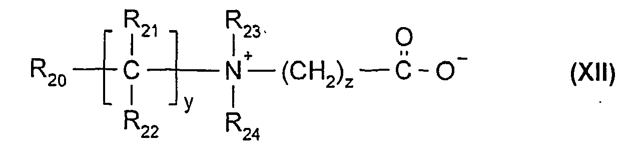 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10. Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle / méthacrylate de diméthylcarboxyméthylammonio-éthyle tel que le produit vendu sous la dénomination DIAFORMER Z301® par la société SANDOZ.
(5) les polymères dérivés du chitosane décrits notamment dans les brevets français N°-2137684 ou US-3879376, comportant des motifs monomères répondant aux formules (XIII), (XIV), (XV) suivantes réunies dans leur chaîne: 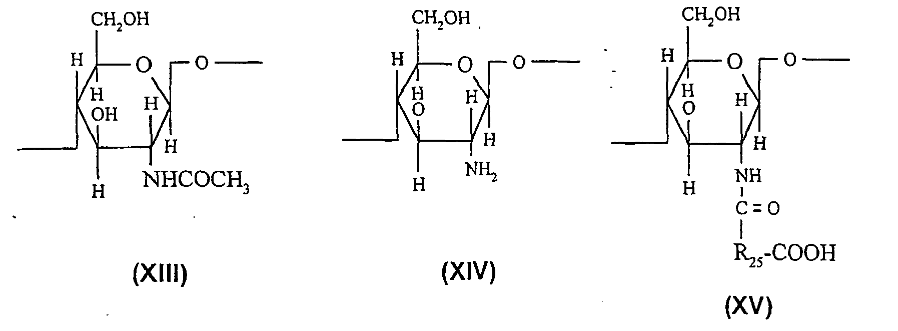 le motif (XIII) étant présent dans des proportions comprises entre 0 et 30%, le motif (XIV) dans des proportions comprises entre 5 et 50% et le motif (XV) dans des proportions comprises entre 30 et 90%, étant entendu que dans ce motif (XV), R₂₅ représente un radical de formule : 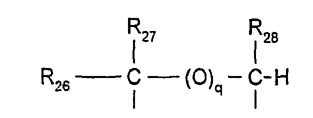 dans laquelle q désigne zéro ou 1 ;
   si q=0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
   Des polymères de ce type plus particulièrement préférés comportent de 0 à 20% en poids de motifs (XIII), de 40 à 50% en poids de motifs (XIV), et de 40 à 50% en poids de motifs (XV) dans lequel R₂₅ désigne le radical -CH₂-CH₂- ;
(6) Les polymères dérivés de la N-carboxyalkylation du chitosane comme le N-carboxyméthyl chitosane ou le N-carboxybutyl chitosane vendu sous la dénomination "EVALSAN®" par la société JAN DEKKER.
(7) Les polymères répondant à la formule générale (XI) tels que ceux décrits par exemple dans le brevet français 1 400 366 : 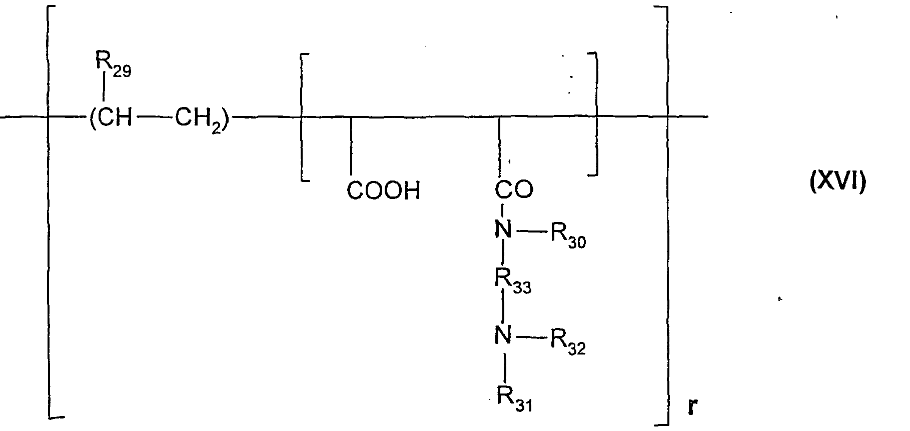 dans laquelle R₂₉ représente un atome d'hydrogène, un radical CH₃O CH₃CH₂O, phényle, R₃₀ désigne l'hydrogène ou un radical alkyle inférieur tel que méthyle, éthyle, R₃₁désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)₂, R₃₃ représentant un groupement -CH₂-CH₂- , -CH₂-CH₂-CH₂-, -CH₂-CH(CH₃)- , R₃₁ ayant les significations mentionnées ci-dessus, ainsi que les homologues supérieurs de ces radicaux et contenant jusqu'à 6 atomes de carbone,
   r est tel que le poids moléculaire est compris entre 500 et 6000000 et,de préférence entre 1000 et 1000000.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (XVII)

      où D désigne un radical 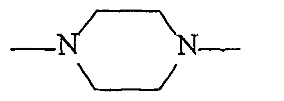 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- (XVIII)
   où D désigne un radical 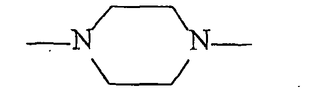 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Parmi tous les polymères amphotères susceptibles d'être utilisés dans le cadre de la présente invention, on préfère mettre en oeuvre les polymères de la famille (1), et en particulier les copolymères d'acide acrylique et de chlorure de diméthyldiallylammonium

Selon l'invention, le ou les polymères cationiques ou amphotères différant des polymères associatifs cationiques ou amphotères et des polymères à motifs (i) acrylamide, (ii)halogénure de dialkyldiallylammonium, et (iii) acide acrylique selon l'invention, peuvent représenter environ 0,01 % à 10 % en poids, de préférence 0,05 % à 5 % en poids, et encore plus préférentiellement 0,1 % à 3 % en poids, du poids total de la composition.

La composition colorante peut encore comprendre une quantité efficace d'autres agents, par ailleurs antérieurement connus en coloration d'oxydation, tels que divers adjuvants usuels comme des séquestrants tel que l'EDTA et l'acide étidronique, des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol, des opacifiants, etc....
La composition colorante peut également comprendre d'autres agents d'ajustement de la rhéologie tels que les épaississants cellulosiques (hydroxyéthycellulose, hydroxypropylcellulose, carboxyméthylcellulose..), la gomme de guar et ses dérivés( hydroxypropylguar..), les gommes d'origine microbienne (gomme de xanthane, gomme de scléroglucane..), les épaississants synthétiques tels que les homopolymères réticulés d'acide acrylique ou d'acide acrylamidopropanesulfonique.
Ces épaississants d'appoint peuvent représenter de 0,01 à 10% en poids du poids total de la composition.

Ladite composition peut également comprendre des agents réducteurs ou antioxydants. Ceux-ci peuvent être choisis en particulier parmi le métabisulfite de sodium, l'acide thioglycolique, l'acide thiolactique, le bisulfite de sodium, l'acide déhydroascorbique, l'hydroquinone, la 2-méthyl-hydroquinone, la ter-butyl-hydroquinone et l'acide homogentisique, et ils sont alors généralement présents dans des quantités allant d'environ 0,05 à 3% en poids par rapport au poids total de la composition.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Dans la composition prête à l'emploi ou dans la composition oxydante, l'agent oxydant est choisi de préférence parmi le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.
On peut également utiliser à titre d'agent oxydant une ou plusieurs enzymes d'oxydoréduction telles que les oxydoréductases à 4 électrons (comme les laccases), les peroxydases et les oxydoréductases à 2 électrons (telles que l'uricase), le cas échéant en présence de leur donneur ou cofacteur respectif.

Le pH de la composition prête à l'emploi et appliquée sur les fibres kératiniques [composition résultant du mélange de la composition tinctoriale et de la composition oxydante], est généralement compris entre les valeurs 3 et 12. Il est de préférence compris entre 8,5 et 11, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VI) suivante : 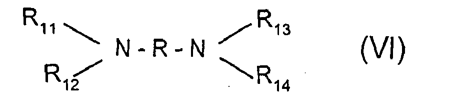 dans laquelle R est un reste propylène éventuellement substitué par un groupement hydroxyle ou un radical alkyle en C₁-C₄ ; R₁₁, R₁₂, R₁₃ et R₁₄, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le procédé de teinture selon l'invention consiste, de préférence, à appliquer un mélange, réalisé extemporanément au moment de l'emploi, (composition prête à l'emploi) à partir des compositions colorante et oxydante décrites ci-avant, sur les fibres kératiniques sèches ou humides, et à le laisser agir pendant un temps de pause variant, de préférence, de 1 à 60 minutes environ, et plus préférentiellement de 5 à 45 minutes environ, à rincer les fibres, puis éventuellement à les laver au shampooing, puis à les rincer à nouveau, et à les sécher.

Des exemples concrets illustrant l'invention vont maintenant être donnés, sans pour autant présenter un caractère limitatif.

### EXEMPLE 1:

On a synthétisé le polyuréthane associatif cationique suivant :

### Réactifs:

- polyoxyde d'éthylène (PEG)(Mₙ 10 000): 0,010 mole
- méthylènedicyclohexyldiisocyanate: 0,018 mole
- N,N-diméthyléthanolamine : 0,020 mole
- bromure de stéaryle : 0,024 mole
- octanoate d'étain (catalyseur) : 0,2 %

On a solubilisé 0,010 mole (100 g) de poly(oxyde d'éthylène) (PEG) ayant une masse moyenne en nombre de 10 000 dans 105 g de THF contenant 0,2 % d'octanoate d'étain (catalyseur), puis on a ajouté goutte à goutte 0,018 mole (4,71 g) de méthylènedicyclohexyldiisocyanate. On a chauffé le milieu réactionnel pendant 15 heures à reflux du THF en ajoutant après 6 heures 100 ml de THF. Pendant la réaction, on a observé une disparition partielle de la bande NCO de l'isocyanate en FTIR et l'apparition des bandes CO et NH des liaisons amide formées. Le milieu était très visqueux et transparent.

On a ajouté ensuite 0,020 moles (1,78 g) de N,N-diméthyléthanolamine et on a laissé la réaction se poursuivre pendant 4 heures au reflux du THF jusqu'à disparition complète de la bande NCO et de la bande OH de l'alcool.

Pour la quaternisation, on a ajouté au mélange réactionnel 0,024 mole (8 g) de bromure de stéaryle, c'est-à-dire un excès de 20 % en moles par rapport à la N,N-diméthyléthanolamine, puis 100 g de THF pour fluidifier le milieu réactionnel très visqueux. On a poursuivi le chauffage au reflux du THF pendant 36 heures supplémentaires.

On a précipité le polymère obtenu dans de l'éther de pétrole, on a filtré et on a séché sous vide à 40 °C pendant 24 heures. On a obtenu ainsi une poudre blanche friable.

On a mesuré par chromatographie par perméation de gel en milieu aqueux (étalonnage avec polystyrène) une masse moyenne en nombre de 70 000 et une masse moyenne en poids de 115 000, ce qui correspondait à un indice de polydispersité de 1,65.

### EXEMPLE 2:

On a préparé la composition de teinture d'oxydation, conforme à l'invention, suivante : (exprimée en grammes)
M.A* désigne Matière Active.

### Composition colorante :

| | |
|---|---|
| Mélange d'alcools linéaires en C18 à C24 [C18/C20/C22/C24 : 7/58/30/6-teneur en alcool > 95%] | 3 |
| Mélange d'alcools linéaires en C18 à C24 [C18/C20/C22/C24 : 7/58/30/6 -teneur en alcool > 95%] oxyéthylénés (30 OE) | 1 |
| Alcool stéarylique oxyéthyléné (2 OE) | 4,5 |
| Alcool stéarylique oxyéthyléné (21 OE) | 1,75 |
| Acide oléique | 2,6 |
| Acide polyacrylique réticulé (Carbopol 980 de GOODRICH) | 0,6 |
| Polyuréthane associatif cationique de l'exemple 1 | 3,5MA* |
| Monoisopropanolamide d'acides de coprah | 3 |
| MERQUAT PLUS 3330 vendu par CALGON en solution aqueuse à 10% MA* | 1,2 MA* |
| Propylène glycol | 6 |
| Métabisulfite de sodium | 0,71 |
| EDTA (Acide éthylènediamine tétra-acétique) | 0,2 |
| Ter-butyl hydroquinone | 0,3 |
| 1,4-diaminobenzène | 0,5 |
| Para-aminophénol | 0,1 |
| 1,3-dihydroxybenzène | 0,6 |
| 1-hydroxy-3-amino-benzène | 0,1 |
| 1-β-hydroxyéthyloxy-2,4-diamino-benzène, dichlorhydrate | 0,04 |
| Monoéthanolamine | 1 |
| Ammoniaque à 20% de NH3 | 11 |
| Parfum q.s | |
| Eau déminéralisée q.s.p | 100 |

### Composition oxydante :

| | |
|---|---|
| Alcool gras | 2,3 |
| Alcool gras oxyéthyléné | 0,6 |
| Amide grasse | 0,9 |
| Glycérine | 0,5 |
| Peroxyde d'hydrogène | 7,5 |
| parfum | qs |
| Eau déminéralisée qsp | 100 |

Ladite composition colorante a été mélangée, au moment de l'emploi, dans un bol en plastique à la composition oxydante donnée ci-dessus, à raison de 1 partie de composition colorante pour 1,5 parties de composition oxydante.
On a appliqué le mélange obtenu sur des mèches de cheveux naturels à 90% de blancs et on a laissé poser 30 minutes.
On a ensuite rincé les mèches à l'eau, on les a lavées au shampooing standard, à nouveau rincées à l'eau, puis séchées et démélées.
Les cheveux ont été teints dans une nuance blond foncé, tenace, et l'état cosmétique du cheveu a été amélioré.

### EXEMPLE 3 :

On a préparé la composition de teinture d'oxydation, conforme à l'invention, suivante : (exprimée en grammes)
M.A* désigne Matière Active

### Composition colorante:

| | |
|---|---|
| Mélange d'alcools linéaires en C18 à C24 [C18/C20/C22/C24 : 7/58/30/6 -teneur en alcool > 95%] | 3 |
| Mélange d'alcools linéaires en C18 à C24 [C18/C20/C22/C24 : 7/58/30/6 -teneur en alcool > 95%] oxyéthylénés (30 OE) | 1 |
| Alcool stéarylique oxyéthyléné (2 OE) | 4,5 |
| Alcool stéarylique oxyéthyléné (21 OE) | 1,75 |
| Acide oléique | 2,6 |
| Acide polyacrylique réticulé (Carbopol 980 de GOODRICH) | 0,6 |
| Aculyn 44 ou Aculyn 46 vendu par ROHM & HAAS | 4 |
| Monoisopropanolamide d'acides de coprah | 3 |
| MERQUAT PLUS 3330 vendu par CALGON en solution aqueuse à 10% MA* | 1,2 MA* |
| Propylène glycol | 6 |
| Métabisulfite de sodium | 0,71 |
| EDTA (Acide éthylènediamine tétra-acétique) | 0,2 |
| Ter-butyl hydroquinone | 0,3 |
| 1,4-diaminobenzène | 0,5 |
| Para-aminophénol | 0,1 |
| 1,3-dihydroxybenzène | 0,6 |
| 1-hydroxy-3-amino-benzène | 0,1 |
| 1-β-hydroxyéthyloxy-2,4-diamino-benzène, dichlorhydrate | 0,04 |
| Monoéthanotamine | 1 |
| Ammoniaque à 20% de NH3 | 11 |
| Parfum q.s | |
| Eau déminéralisée q.s.p | 100 |

### Composition oxydante :

| | |
|---|---|
| Alcool gras | 2,3 |
| Alcool gras oxyéthyléné | 0,6 |
| Amide grasse | 0,9 |
| Glycérine | 0,5 |
| Peroxyde d'hydrogène | 7,5 |
| parfum | qs |
| Eau déminéralisée qsp | 100 |

Ladite composition colorante a été mélangée, au moment de l'emploi, dans un bol en plastique à la composition oxydante donnée ci-dessus, à raison de 1 partie de composition colorante pour 1,5 parties de composition oxydante.
On a appliqué le mélange obtenu sur des mèches de cheveux naturels à 90% de blancs et on a laissé poser 30 minutes.
On a ensuite rincé les mèches à l'eau, on les a lavées au shampooing standard, à nouveau rincées à l'eau, puis séchées et démélées.
Les cheveux ont été teints dans une nuance blond foncé, tenace, et l'état cosmétique du cheveu a été amélioré.

### EXEMPLE 4:

On a reproduit la composition de l'exemple 3, en remplaçant les 4 grammes d'Aculyn 44® ou d'Aculyn 46® par 3,8g MA de Quatrisoft LM200® vendu par AMERCHOL. En suivant le même protocole qu'à l'exemple 3, la composition de teinture d'oxydation obtenue a donné des performances identiques en terme d'état cosmétique du cheveu et de puissance de la teinture.

## Revendications

1. Composition pour la teinture des fibres kératiniques, en particulier des fibres kératiniques humaines, et plus particulièrement les cheveux, comprenant, dans un milieu approprié pour la teinture, au moins un colorant et au moins un polymère associatif non ionique, cationique ou amphotère, et qui est **caractérisée par le fait qu'**elle contient en outre au moins un polymère à motifs (i) acrylamide, (ii)halogénure de dialkyldiallylammonium, et (iii) acide carboxylique vinylique.

2. Composition selon la revendication 1, **caractérisée par le fait que** le colorant est un colorant direct ou un colorant d'oxydation.

3. Composition selon la revendication 2, **caractérisée par le fait que** le ou les colorants d'oxydation sont choisis parmi les bases d'oxydation et/ou les coupleurs.

4. Composition selon la revendication 3, **caractérisée par le fait que** les bases d'oxydation sont choisies parmi les ortho- ou para-phénylènediamines, les bases doubles, les ortho- ou para-aminophénols, et les bases hétérocycliques, ainsi que les sels d'addition de ces composés avec un acide, et sont présentes dans des concentrations allant de 0,0005 à 12% en poids par rapport au poids total de la composition.

5. Composition selon la revendication 3, **caractérisée par le fait que** les coupleurs sont choisis parmi les métaaminophénols, les métaphénylènediamines, les métadiphénols, les naphtols, les coupleurs hétérocycliques, et les sels d'addition de ces composés avec un acide, et sont présents dans des concentrations allant de 0,0001 à 10% en poids par rapport au poids total de la composition.

6. Composition selon l'une quelconque des revendications 4 ou 5, **caractérisée par le fait que** les sels d'addition avec un acide des colorants d'oxydation (bases et coupleurs) sont choisis parmi les chlorhydrates, les bromhydrates, les sulfates, les tartrates, les lactates et les acétates.

7. Composition selon la revendication 3, **caractérisée par le fait qu'**elle renferme en outre au moins un agent oxydant.

8. Composition selon la revendication 7, **caractérisée par le fait qu'**elle contient en outre au moins un colorant direct dans la proportion de 0,001 à 20% en poids par rapport au poids total de la composition.

9. Composition selon l'une quelconque des revendications précédentes, **caractérisée par le fait que** dans le polymère à motifs (i) acrylamide, (ii)halogénure de dialkyldiallylammonium, et (iii) acide carboxylique vinylique, le motif acrylamide est choisi parmi les motifs de structure (1) suivante: 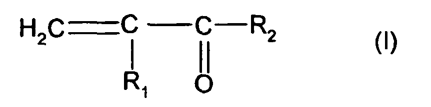 dans laquelle,
- R₁ désigne H ou CH₃,
- R₂ est choisi parmi un radical amino, diméthylamino, tert-butylamino, dodécylamino, ou -NH-CH₂OH.

10. Composition selon la revendication 9, **caractérisée par** le fait dans la formule (I) R₁ est hydrogène et R₂ est un radical amino.

11. Composition selon l'une quelconque des revendications précédentes, **caractérisée par le fait que** dans le polymère à motifs (i) acrylamide, (ii)halogénure de dialkyldiallylammonium, et (iii) acide carboxylique vinylique, le motif halogénure de dialkyldiallylammonium est le chlorure de diméthyldiallylammonium.

12. Composition selon l'une quelconque des revendications précédentes, **caractérisée par le fait que** dans le polymère à motifs (i) acrylamide, (ii)halogénure de dialkyldiallylammonium, et (iii) acide carboxylique vinylique, le motif acide carboxylique vinylique est choisi parmi l'acide acrylique, l'acide méthacrylique, et l'acide 2-acrylamido-2-méthyl-propane sulfonique.

13. Composition selon la revendication 12, **caractérisée par** le fait le motif (iii) est l'acide acrylique.

14. Composition selon l'une quelconque des revendications précédentes, **caractérisée par le fait que** le polymère à motifs (i) acrylamide, (ii)halogénure de dialkyldiallylammonium, et (iii) acide carboxylique vinylique est le terpolymère acrylamide / chlorure de diméthyldiallylammonium / acide acrylique.

15. Composition selon l'une quelconque des revendications précédentes, **caractérisée par le fait que** le ou les polymères à motifs (i) acrylamide, (ii)halogénure de dialkyldiallylammonium, et (iii) acide carboxylique vinylique sont présents dans la proportion de 0,1 à 10% en poids par rapport au poids total de la composition.

16. Composition selon la revendication 15, **caractérisée par le fait que** le ou les polymères à motifs (i) acrylamide, (ii)halogénure de dialkyldiallylammonium, et (iii) acide carboxylique vinylique sont présents dans la proportion de 0,5 à 5% en poids par rapport au poids total de la composition.

17. Composition selon la revendication 16, **caractérisée par le fait que** le ou les polymères à motifs (i) acrylamide, (ii)halogénure de dialkyldiallylammonium, et (iii) acide carboxylique vinylique sont présents dans la proportion de 1 à 3% en poids par rapport au poids total de la composition.

18. Composition selon la revendication 1, **caractérisée par le fait que** le ou les polymères associatifs sont non ioniques et choisis parmi les polyéthers polyuréthanes.

19. Composition selon la revendication 18, **caractérisée par le fait que** le polyéther polyuréthane est un polycondensat de polyéthylèneglycol à 150 ou 180 moles d'oxyde d'éthylène, d'alcool stéarylique et d'au moins un diisocyanate.

20. Composition selon la revendication 19, **caractérisée par le fait qu'**il s'agit d'un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%).

21. Composition selon la revendication 18, **caractérisée par le fait que** le polyéther polyuréthane est un polycondensat de polyéthylèneglycol à 150 ou 180 moles d'oxyde d'éthylène, d'alcool décylique et d'au moins un diisocyanate.

22. Composition selon la revendication 21, **caractérisée par le fait que** le polyéther polyuréthane est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

23. Composition selon la revendication 1, **caractérisée par le fait que** le ou les polymères associatifs cationiques sont choisis parmi :
- (I) les polyuréthanes associatifs cationiques représentées par la formule générale (la) suivante :
R-X-(P)ₙ-[L-(Y)ₘ]ᵣ-L'-(P')ₚ-X'-R' (Ia)
dans laquelle :
R et R', identiques ou différents, représentent un groupement hydrophobe ou un atome d'hydrogène ;
X et X', identiques ou différents, représentent un groupement comportant une fonction amine portant ou non un groupement hydrophobe, ou encore le groupement L" ;
L, L' et L", identiques ou différents, représentent un groupement dérivé d'un diisocyanate;
P et P', identiques ou différents, représentent un groupement comportant une fonction amine portant ou non un groupement hydrophobe ;
Y représente un groupement hydrophile ;
r est un nombre entier compris entre 1 et 100, de préférence entre 1 et 50 et en particulier entre 1 et 25,
n, m, et p valent chacun indépendamment des autres entre 0 et 1000 ;
la molécule contenant au moins une fonction amine protonée ou quaternisée et au moins un groupement hydrophobe;
- (II) les dérivés de cellulose quaternisée et les polyacrylates à groupements latéraux aminés non cycliques.

24. Composition selon la revendication 1, **caractérisée par le fait que** le ou les polymères associatifs amphotères comprennent, ou sont préparés en copolymérisant :
1) au moins un monomère de formule (la) ou (Ib): 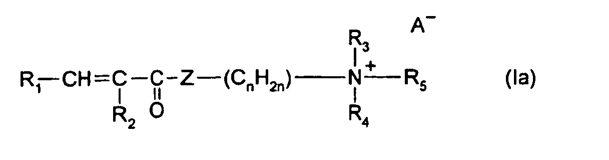 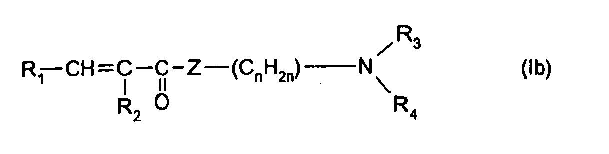 dans lesquelles, R₁ et R₂, identiques ou différents, représentent un atome d'hydrogène ou un radical méthyle, R₃, R₄ et R₅, identiques ou différents, représente un radical alkyle linéaire ou ramifié ayant de 1 à 30 atomes de carbone,
Z représente un groupe NH ou un atome d'oxygène,
n est un nombre entier de 2 à 5,
A⁻ est un anion issu d'un acide organique ou minéral, tel qu'un anion méthosulfate ou un halogénure tel que chlorure ou bromure;
2) au moins un monomère de formule (II)
R₆-CH= CR₇-COOH (II)
dans laquelle, R₆ et R₇, identiques ou différents, représentent un atome d'hydrogène ou un radical méthyle; et
3) au moins un monomère de formule (III) :
R₆-CH= CR₇- COXR₈ (III)
dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone ; l'un au moins des monomères de formule (Ia), (Ib) ou (III) comportant au moins une chaîne grasse. 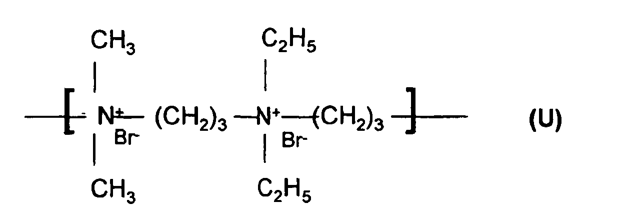
-3/ les polymères aux motifs de formule (IX) suivante : 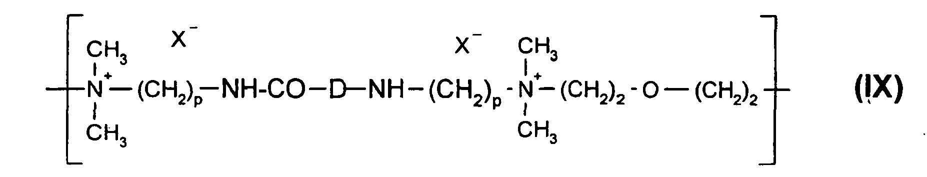 pour laquelle, p est égal à 3, et,
a) D désigne la valeur zéro, X désigne un atome de chlore,
b) D représente un groupement -(CH₂)₄ -CO -, X désigne un atome de chlore,
c) D représente un groupement -(CH₂)₇ -CO-, X désigne un atome de chlore,
d) un " Block Copolymer " formé de motifs correspondant aux polymères décrits aux alinéas a) et b);
-4/ les copolymères d'acide acrylique et de chlorure de diméthyldiallylammonium.

25. Composition selon l'une quelconque des revendications 17 à 24, **caractérisée par le fait que** le ou les polymères associatifs sont présents dans la proportion de 0,1 à 10% en poids par rapport au poids total de la composition.

26. Composition selon la revendication 25, **caractérisée par le fait que** le ou les polymères associatifs sont présents dans la proportion de 0,5 à 5% en poids par rapport au poids total de la composition.

27. Composition selon la revendication 26, **caractérisée par le fait que** le ou les polymères associatifs sont présents dans la proportion de 1 à 3% en poids par rapport au poids total de la composition.

28. Composition selon l'une quelconque des revendications précédentes, **caractérisée par le fait qu'**elle contient en outre au moins un polymère cationique ou amphotère différent des polymères associatifs et des polymères à motifs (i) acrylamide, (ii)halogénure de dialkyldiallylammonium, et (iii) acide carboxylique vinylique décrits à l'une quelconque des revendications précédentes.

29. Composition selon la revendication 28, **caractérisée par le fait que** le polymère cationique ou amphotère est choisi parmi:
-1/ les homopolymères de chlorure de diméthyldiallylammonium;
-2/ les polymères au motifs récurrents de formules (W) ou (U) suivantes:

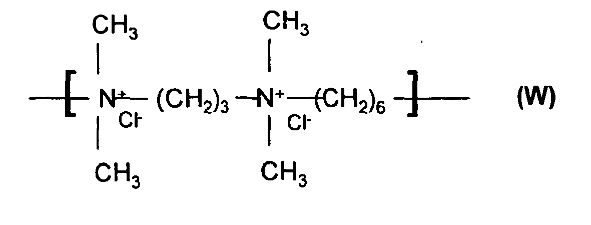

30. Composition selon la revendication 28 ou 29, **caractérisée par le fait que** le ou lesdits polymères cationiques ou amphotères sont présents dans la proportion de 0,01 à 10% en poids par rapport au poids total de la composition.

31. Composition selon l'une quelconque des revendications précédentes, **caractérisée par le fait qu'**elle contient au moins un agent tensioactif choisi parmi les tensioactifs anioniques, cationiques, non ioniques ou amphotères ou zwittérioniques dans la proportion d'au moins 0,01 % en poids par rapport au poids total de la composition.

32. Composition selon la revendication 31, **caractérisée par le fait que** l'agent tensio-actif est non ionique.

33. Composition selon la revendication 7, **caractérisée par le fait qu'**elle possède un pH compris entre 3 et 12.

34. Composition selon la revendication 33, **caractérisée par le fait que** le pH est compris entre 8,5 et 11.

35. Composition selon la revendication 7, **caractérisée par le fait que** l'agent oxydant est choisi parmi le peroxyde d'hydrogène, le peroxyde d'urée, les bromates ou ferricyanures de métaux alcalins, les persels, les enzymes d'oxydoréduction avec éventuellement leur donneur ou cofacteur respectif.

36. Composition selon la revendication 35, **caractérisée par le fait que** l'agent oxydant est le peroxyde d'hydrogène.

37. Composition selon la revendication 35, **caractérisée par le fait que** l'agent oxydant est une solution d'eau oxygénée dont le titre varie de 1 à 40 volumes.

38. Procédé de teinture des fibres kératiniques, en particulier des fibres kératiniques humaines et plus particulièrement les cheveux, **caractérisé par le fait qu'**il consiste à appliquer sur les fibres au moins une composition colorante comprenant, dans un milieu approprié pour la teinture, au moins un colorant (direct ou d'oxydation), la couleur étant révélée à pH alcalin, neutre ou acide à l'aide d'une composition oxydante contenant au moins un agent oxydant, qui est mélangée juste au moment de l'emploi à la composition colorante ou qui est appliquée séquentiellement sans rinçage intermédiaire, au moins un polymère associatif non ionique, cationique ou amphotère et au moins un polymère à motifs (i) acrylamide, (ii)halogénure de dialkyldiallylammonium, et (iii) acide carboxylique vinylique défini selon les revendications 9 à 17 étant présents dans la composition colorante ou dans la composition oxydante ou dans chacune desdites compositions.

39. Procédé selon la revendication 38, **caractérisé par le fait que** le ou les polymères associatifs et le ou les polymères à motifs (i) acrylamide, (ii)halogénure de dialkyldiallylammonium, et (iii) acide carboxylique vinylique sont présents dans la composition colorante.

40. Procédé selon les revendications 38 ou 39, **caractérisé par le fait qu'**il consiste à appliquer sur les fibres kératiniques sèches ou humides, la composition prête à l'emploi, réalisée extemporanément au moment de l'emploi à partir des compositions colorante et oxydante, à la laisser agir pendant un temps de pause variant de 1 à 60 minutes environ, et de préférence de 5 à 45 minutes, à rincer les fibres, puis éventuellement à les laver au shampooing, puis à les rincer à nouveau, et à les sécher.

41. Dispositif à plusieurs compartiments ou « Kit » pour la teinture des fibres kératiniques, en particulier des fibres kératiniques humaines et plus particulièrement les cheveux, **caractérisé par le fait qu'**il comporte au moins deux compartiments, dont l'un d'entre eux renferme une composition colorante comprenant, dans un milieu approprié pour la teinture, au moins un colorant (direct ou d'oxydation), et un autre une composition oxydante comprenant, dans un milieu approprié pour la teinture, un agent oxydant, au moins un polymère associatif non ionique, cationique ou amphotère et au moins un polymère à motifs (i) acrylamide, (ii)halogénure de dialkyldiallylammonium, et (iii) acide carboxylique vinylique défini selon les revendications 9 à 17 étant présents dans la composition colorante ou dans la composition oxydante ou dans chacune desdites compositions.

## Claims

1. Composition for the dyeing of keratin fibers, in particular of human keratin fibers and more particularly the hair, comprising, in a medium that is suitable for dyeing, at least one dye and at least one nonionic, cationic or amphoteric associative polymer, and which is **characterized in that** it also contains at least one polymer containing (i) acrylamide, (ii) dialkyldiallylammonium halide and (iii) vinylcarboxylic acid units.

2. Composition according to Claim 1, **characterized in that** the dye is a direct dye or an oxidation dye.

3. Composition according to Claim 2, **characterized in that** the oxidation dye(s) is(are) chosen from oxidation bases and/or couplers.

4. Composition according to Claim 3, **characterized in that** the oxidation bases are chosen from ortho- or para-phenylenediamines, double bases, ortho- or para-aminophenols and heterocyclic bases, and also the addition salts of these compounds with an acid, and are present in concentrations ranging from 0.0005% to 12% by weight relative to the total weight of the composition.

5. Composition according to Claim 3, **characterized in that** the couplers are chosen from meta-aminophenols, meta-phenylenediamines, meta-diphenols, naphthols and heterocyclic couplers, and the addition salts of these compounds with an acid, and are present in concentrations ranging from 0.0001% to 10% by weight relative to the total weight of the composition.

6. Composition according to either of Claims 4 and 5, **characterized in that** the addition salts with an acid of the oxidation dyes (bases and couplers) are chosen from the hydrochlorides, hydrobromides, sulfates, tartrates, lactates and acetates.

7. Composition according to Claim 3, **characterized in that** it also contains at least one oxidizing agent.

8. Composition according to Claim 7, **characterized in that** it also contains at least one direct dye in a proportion of from 0.001% to 20% by weight relative to the total weight of the composition.

9. Composition according to any one of the preceding claims, **characterized in that**, in the polymer containing (i) acrylamide, (ii) dialkyldiallylammonium halide and (iii) vinylcarboxylic acid units, the acrylamide unit is chosen from the units of structure (I) below: 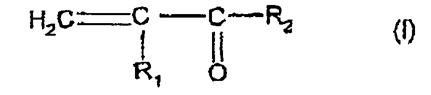 in which:
- R₁ denotes H or CH₃,
- R₂ is chosen from an amino, dimethylamino, tert-butylamino, dodecylamino or -NH-CH₂OH radical.

10. Composition according to Claim 9, **characterized in that**, in formula (I), R₁ is hydrogen and R₂ is an amino radical.

11. Composition according to any one of the preceding claims, **characterized in that**, in the polymer containing (i) acrylamide, (ii) dialkyldiallylammonium halide and (iii) vinylcarboxylic acid units, the dialkyldiallylammonium halide unit is dimethyldiallylammonium chloride.

12. Composition according to any one of the preceding claims, **characterized in that**, in the polymer containing (i) acrylamide, (ii) dialkyldiallylammonium halide and (iii) vinylcarboxylic acid units, the vinylcarboxylic acid unit is chosen from acrylic acid, methacrylic acid and 2-acrylamido-2-methylpropanesulfonic acid.

13. Composition according to Claim 12, **characterized in that** the unit (iii) is acrylic acid.

14. Composition according to any one of the preceding claims, **characterized in that** the polymer containing (i) acrylamide, (ii) dialkyldiallylammonium halide and (iii) vinylcarboxylic acid units is the acrylamide/dimethyldiallylammonium chloride/acrylic acid terpolymer.

15. Composition according to any one of the preceding claims, **characterized in that** the polymer(s) containing (i) acrylamide, (ii) dialkyldiallylammonium halide and (iii) vinylcarboxylic acid units is(are) present in a proportion of from 0.1% to 10% by weight relative to the total weight of the composition.

16. Composition according to Claim 15, **characterized in that** the polymer(s) containing (i) acrylamide, (ii) dialkyldiallylammonium halide and (iii) vinylcarboxylic acid units is(are) present in a proportion of from 0.5% to 5% by weight relative to the total weight of the composition.

17. Composition according to Claim 16, **characterized in that** the polymer(s) containing (i) acrylamide, (ii) dialkyldiallylammonium halide and (iii) vinylcarboxylic acid units is(are) present in a proportion of from 1% to 3% by weight relative to the total weight of the composition.

18. Composition according to Claim 1, **characterized in that** the associative polymer(s) is (are) nonionic and chosen from polyurethane polyethers.

19. Composition according to Claim 18, **characterized in that** the polyurethane polyether is a polycondensate of polyethylene glycol containing 150 or 180 mol of ethylene oxide, of stearyl alcohol and of at least one diisocyanate.

20. Composition according to Claim 19, **characterized in that** it is a polycondensate of polyethylene glycol containing 150 or 180 mol of ethylene oxide, of stearyl alcohol and of methylenebis(4-cyclohexyl isocyanate) (SMDI), at 15% by weight in a matrix of maltodextrin (4%) and water (81%).

21. Composition according to Claim 18, **characterized in that** the polyurethane polyether is a polycondensate of polyethylene glycol containing 150 or 180 mol of ethylene oxide, of decyl alcohol and of at least one diisocyanate.

22. Composition according to Claim 21, **characterized in that** the polyurethane polyether is a polycondensate of polyethylene glycol containing 150 or 180 mol of ethylene oxide, of decyl alcohol and of methylenebis(4-cyclohexyl isocyanate) (SMDI), at 35% by weight in a mixture of propylene glycol (39%) and water (26%).

23. Composition according to Claim 1,
**characterized in that** the cationic associative polymer or polymers are chosen from:
- (I) the cationic associative polyurethanes represented by the general formula (Ia) below: R-X-(P)ₙ-[L-(Y)ₘ]ᵣ-L'-(P')ₚ-X'-R' (Ia) in which:
R and R', which may be identical or different, represent a hydrophobic group or a hydrogen atom;
X and X', which may be identical or different, represent a group comprising an amine function optionally bearing a hydrophobic group, or alternatively a group L";
L, L' and L", which may be identical or different, represent a group derived from a diisocyanate;
P and P', which may be identical or different, represent a group comprising an amine function optionally bearing a hydrophobic group;
Y represents a hydrophilic group;
r is an integer between 1 and 100, preferably between 1 and 50 and in particular between 1 and 25,
n, m and p each range, independently of each other, from 0 to 1000;
the molecule containing at least one protonated or quaternized amine function and at least one hydrophobic group;
- (II) quaternized cellulose derivatives and polyacrylates containing non-cyclic amino side groups.

24. Composition according to Claim 1, **characterized in that** the amphoteric associative polymer or polymers comprise, or are prepared by copolymerizing:
1) at least one monomer of formula (Ia) or (Ib) : 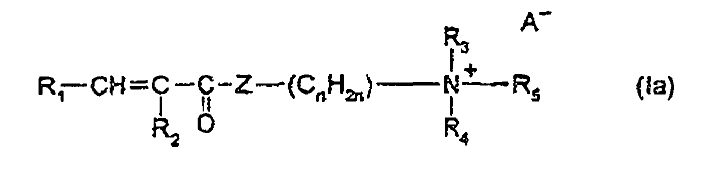 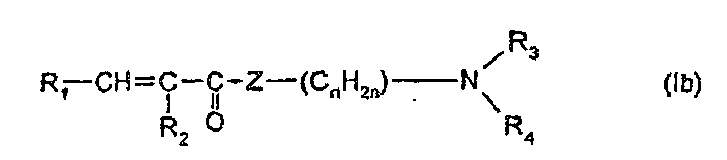 in which R₁ and R₂, which may be identical or different, represent a hydrogen atom or a methyl radical, R₃, R₄ and R₅, which may be identical or different, represent a linear or branched alkyl radical containing from 1 to 30 carbon atoms,
Z represents an NH group or an oxygen atom,
n is an integer from 2 to 5,
A⁻ is an anion derived from an organic or mineral acid, such as a methosulfate anion or a halide such as chloride or bromide;
2) at least one monomer of formula (II)
R₆-CH= CR₇-COOH (II)
in which R₆ and R₇, which may be identical or different, represent a hydrogen atom or a methyl radical; and
3) at least one monomer of formula (III):
R₆-CH= CR₇- COXR₈ (III)
in which R₆ and R₇, which may be identical or different, represent a hydrogen atom or a methyl radical, X denotes an oxygen or nitrogen atom and R₈ denotes a linear or branched alkyl radical containing from 1 to 30 carbon atoms; at least one of the monomers of formula (Ia), (Ib) or (III) comprising at least one fatty chain.

25. Composition according to any one of Claims 17 to 24, **characterized in that** the associative polymer(s) is (are) present in a proportion of from 0.1% to 10% by weight relative to the total weight of the composition.

26. Composition according to Claim 25, **characterized in that** the associative polymer(s) is (are) present in a proportion of from 0.5% to 5% by weight relative to the total weight of the composition.

27. Composition according to Claim 26, **characterized in that** the associative polymer(s) is(are) present in a proportion of from 1% to 3% by weight relative to the total weight of the composition.

28. Composition according to any one of the preceding claims, **characterized in that** it also contains at least one cationic or amphoteric polymer other than the associative polymers and than the polymers containing (i) acrylamide, (ii) dialkyldiallylammonium halide and (iii) vinylcarboxylic acid units described in any one of the preceding claims.

29. Composition according to Claim 28, **characterized in that** the cationic or amphoteric polymer is chosen from:
-1/ dimethyldiallylammonium chloride homopolymers;
-2/ polymers containing the repeating units of formula (W) or (U) below: 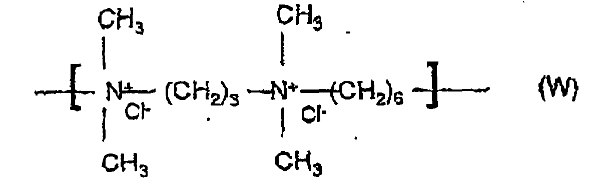 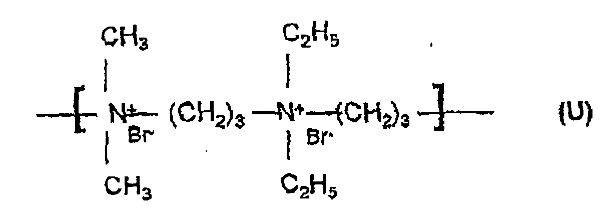
-3/ polymers containing the units of formula (IX) below: 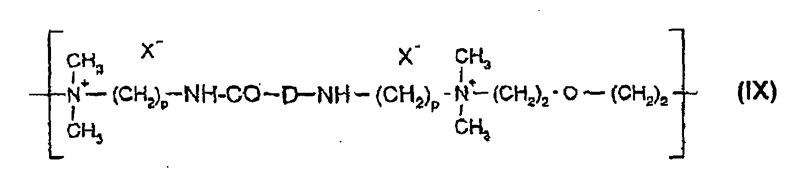 for which p is equal to 3, and
a) D denotes the value zero, X denotes a chlorine atom,
b) D represents a -(CH₂)₄-CO- group, X denotes a chlorine atom,
c) D represents a -(CH₂)₇-CO- group, X denotes a chlorine atom,
d) a "Block Copolymer" formed from units corresponding to the polymers described in paragraphs a) and b);
-4/ copolymers of acrylic acid and of dimethyldiallylammonium chloride.

30. Composition according to Claim 28 or 29, **characterized in that** said cationic or amphoteric polymer(s) is(are) present in a proportion of from 0.01% to 10% by weight relative to the total weight of the composition.

31. Composition according to any one of the preceding claims, **characterized in that** it contains at least one surfactant chosen from anionic, cationic, nonionic, amphoteric and zwitterionic surfactants, in a proportion of at least 0.01% by weight relative to the total weight of the composition.

32. Composition according to Claim 31, **characterized in that** the surfactant is nonionic.

33. Composition according to Claim 7, **characterized in that** it has a pH of between 3 and 12.

34. Composition according to Claim 33, **characterized in that** the pH is between 8.5 and 11.

35. Composition according to Claim 7, **characterized in that** the oxidizing agent is chosen from hydrogen peroxide, urea peroxide, alkali metal bromates or ferricyanides, persalts, and redox enzymes optionally with their respective donor or co-factor.

36. Composition according to Claim 35, **characterized in that** the oxidizing agent is hydrogen peroxide.

37. Composition according to Claim 35, **characterized in that** the oxidizing agent is an aqueous hydrogen peroxide solution whose titer ranges from 1 to 40 volumes.

38. Process for the dyeing of keratin fibers, in particular of human keratin fibers and more particularly the hair, **characterized in that** it consists in applying to the fibers at least one dye composition comprising, in a medium that is suitable for dyeing, at least one dye (direct dye or oxidation dye), the color being developed at alkaline, neutral or acidic pH using an oxidizing composition containing at least one oxidizing agent, which is mixed with the dye composition just at the time of use or which is applied sequentially without intermediate rinsing, at least one nonionic, cationic or amphoteric associative polymer and at least one polymer containing (i) acrylamide, (ii) dialkyldiallylammonium halide and (iii) vinylcarboxylic acid units defined according to Claims 9 to 17 being present in the dye composition or in the oxidizing composition or in each of said compositions.

39. Process according to Claim 38, **characterized in that** the associative polymer(s) and the polymer(s) containing (i) acrylamide, (ii) dialkyldiallylammonium halide and (iii) vinylcarboxylic acid units are present in the dye composition.

40. Process according to Claim 38 or 39, **characterized in that** it consists in applying to wet or dry keratin fibers the ready-to-use composition prepared extemporaneously at the time of use from the dye composition and oxidizing composition, in leaving the composition to act for an exposure time ranging from 1 to 60 minutes approximately, and preferably from 5 to 45 minutes, in rinsing the fibers and then in optionally washing them with shampoo, then rinsing them again and drying them.

41. Multi-compartment device or "kit" for the dyeing of keratin fibers, in particular of human keratin fibers and more particularly the hair, **characterized in that** it comprises at least two compartments, one of which contains a dye composition comprising, in a medium that is suitable for dyeing, at least one dye (direct dye or oxidation dye), and another containing an oxidizing composition comprising, in a medium that is suitable for dyeing, an oxidizing agent, at least one nonionic, cationic or amphoteric associative polymer and at least one polymer containing (i) acrylamide, (ii) dialkyldiallylammonium halide and (iii) vinylcarboxylic acid units defined according to Claims 9 to 17 being present in the dye composition or in the oxidizing composition or in each of said compositions.

## Patentansprüche

1. Zusammensetzung zum Färben von Keratinfasern und insbesondere menschlichen Keratinfasern, besonders Haaren, die in einem zum Färben geeigneten Medium mindestens ein Farbmittel und mindestens ein nichtionisches, kationisches oder amphoteres assoziatives Polymer enthält, **dadurch gekennzeichnet, dass** sie ferner mindestens ein Polymer mit den Einheiten (i) Acrylamid, (ü) Dialkyldiallylammoniumhalogenid und (iii) Vinylcarbonsäure enthält.

2. Zusammensetzung nach Anspruch 1, **dadurch gekennzeichnet, dass** es sich bei dem Farbmittel um einen Direktfarbstoff oder einen Oxidationsfarbstoff handelt.

3. Zusammensetzung nach Anspruch 2, **dadurch gekennzeichnet, dass** der oder die Oxidationsfarbstoffe unter den Oxidationsbasen und/oder Kupplern ausgewählt sind.

4. Zusammensetzung nach Anspruch 3, **dadurch gekennzeichnet, dass** die Oxidationsbasen unter den o- oder p-Phenylendiaminen, Doppelbasen, *o*- oder *p*-Aminophenolen und heterocyclischen Basen sowie den Additionssalzen dieser Verbindungen mit einer Säure ausgewählt sind und Konzentrationen von 0,0005 bis 12 Gew.-%, bezogen auf das Gesamtgewicht der Zusammensetzung, enthalten sind.

5. Zusammensetzung nach Anspruch 3, **dadurch gekennzeichnet, dass** die Kuppler unter den *m*-Aminophenolen, *m*-Phenylendiaminen, *m*-Dihydroxybenzolen, Naphtholen, heterocyclischen Kupplern und den Additionssalzen dieser Verbindungen mit einer Säure ausgewählt sind und in Konzentrationen von 0,0001 bis 10 Gew.-%, bezogen auf das Gesamtgewicht der Zusammensetzung, enthalten sind.

6. Zusammensetzung nach einem der Ansprüche 4 oder 5, **dadurch gekennzeichnet, dass** die Additionssalze mit Oxidationsfarbstoffen (Basen und Kuppler) mit einer Säure unter den Hydrochloriden, Hydrobromiden, Sulfaten, Tartraten, Lactaten und Acetaten ausgewählt sind.

7. Zusammensetzung nach Anspruch 3, **dadurch gekennzeichnet, dass** sie ferner mindestens ein Oxidationsmittel enthält.

8. Zusammensetzung nach Anspruch 7, **dadurch gekennzeichnet, dass** sie ferner einen Direktfarbstoff in einem Mengenanteil von 0,001 bis 20 Gew.-%, bezogen auf das Gesamtgewicht der Zusammensetzung, enthält.

9. Zusammensetzung nach einem der vorhergehenden Ansprüche, **dadurch gekennzeichnet, dass** in dem Polymer mit den Einheiten (i) Acrylamid, (ii) Dialkyldiallylammoniumhalogenid und (iii) Vinylcarbonsäure die Acrylamideinheit unter den Einheiten der folgenden Struktur (I) ausgewählt ist: 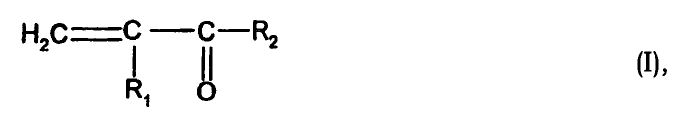 worin
- R₁ H oder CH₃ bedeutet, und
- R₂ unter Amino, Dimethylamino, t-Butylamino, Dodecylamino oder -NH-CH₂OH ausgewählt ist.

10. Zusammensetzung nach Anspruch 9, **dadurch gekennzeichnet, dass** in der Formel (I) R₁ Wasserstoff und R₂ eine Aminogruppe bedeutet.

11. Zusammensetzung nach einem der vorhergehenden Ansprüche, **dadurch gekennzeichnet, dass** in dem Polymer mit den Einheiten (i) Acrylamid, (ii) Dialkyldiallylammoniumhalogenid und (iii) Vinylcarbonsäure die Dialkyldiallylammoniumhalogenideinheit das Dimethyldiallylammoniumchlorid ist.

12. Zusammensetzung nach einem der vorhergehenden Ansprüche, **dadurch gekennzeichnet, dass** in dem Polymer mit Einheiten (i) Acrylamid, (ii) Dialkyldiallylammoniumhalogenid und (iii) Vinylcarbonsäure die Vinylcarbonsäureeinheit unter Acrylsäure, Methacrylsäure und 2-Acrylamido-2-methylpropansulfonsäure ausgewählt ist.

13. Zusammensetzung nach Anspruch 12, **dadurch gekennzeichnet, dass** es sich bei der Einheit (iii) um Acrylsäure handelt.

14. Zusammensetzung nach einem der vorhergehenden Ansprüche, **dadurch gekennzeichnet, dass** das Polymer mit Einheiten (i) Acrylamid, (ii) Dialkyldiallylammoniumhalogenid und (iii) Vinylcarbonsäure das Terpolymer Acrylamid/Dimethyldiallylammoniumchlorid/Acrylsäure ist.

15. Zusammensetzung nach einem der vorhergehenden Ansprüche, **dadurch gekennzeichnet, dass** das oder die Polymere mit Einheiten (i) Acrylamid, (ii) Dialkyldiallylammoniumhalogenid und (iii) Vinylcarbonsäure in Mengenanteilen von 0,1 bis 10 Gew.-%, bezogen auf das Gesamtgewicht der Zusammensetzung, enthalten sind.

16. Zusammensetzung nach Anspruch 15, **dadurch gekennzeichnet, dass** das oder die Polymere mit Einheiten (i) Acrylamid, (ii) Dialkyldiallylammoniumhalogenid und (iii) Vinylcarbonsäure in einem Mengenanteil von 0,5 bis 5 Gew.-%, bezogen auf das Gesamtgewicht der Zusammensetzung, enthalten sind.

17. Zusammensetzung nach Anspruch 16, **dadurch gekennzeichnet, dass** das oder die Polymere mit Einheiten (i) Acrylamid, (ii) Dialkyldiallylammoniumhalogenid und (iii) Vinylcarbonsäure in einem Mengenanteil von 1 bis 3 Gew.-%, bezogen auf das Gesamtgewicht der Zusammensetzung, enthalten sind.

18. Zusammensetzung nach Anspruch 1, **dadurch gekennzeichnet, dass** das oder die assoziativen Polymere nichtionisch und unter den Polyetherpolyurethanen ausgewählt sind.

19. Zusammensetzung nach Anspruch 18, **dadurch gekennzeichnet, dass** das Polyetherpolyurethan ein Polykondensat von Polyethylenglycol mit 150 oder 180 mol Ethylenoxideinheiten, Stearylalkohol und mindestens einem Diisocyanat ist.

20. Zusammensetzung nach Anspruch 19, **dadurch gekennzeichnet, dass** es sich um ein Polykondensat von Polyethylenglycol mit 150 oder 180 mol Ethylenoxid, Stearylalkohol und Methylen-bis(4-cyclohexylisocyanat) (SMDI) von 15 Gew.-% in einer Matrix aus Maltodextrin (4 %) und Wasser (81 %) handelt.

21. Zusammensetzung nach Anspruch 18, **dadurch gekennzeichnet, dass** das Polyetherpolyurethan ein Polykondensat von Polyethylenglycol mit 150 oder 180 mol Ethylenoxid, Decylalkohol und mindestens einem Diisocyanat ist.

22. Zusammensetzung nach Anspruch 21, **dadurch gekennzeichnet, dass** das Polyetherpolyurethan ein Polykondensat von Polyethylenglycol mit 150 oder 180 mol Ethylenoxid, Decylalkohol und Methylen-bis(4-cyclohexylisocyanat) (SMDI) von 35 Gew.-% in einem Gemisch aus Propylenglycol (39 %) und Wasser (26 %) ist.

23. Zusammensetzung nach Anspruch 1, **dadurch gekennzeichnet, dass** das oder die kationischen assoziativen Polymere ausgewählt sind unter:
- (I) kationischen assoziativen Polyurethanen, die die folgende allgemeine Formel (Ia) aufweisen:
R-X-(P)ₙ-[L-(Y)ₘ]ᵣ-L'-(P')ₚ-X'-R' (Ia),
worin bedeuten:
die Gruppen R und R', die gleich oder verschieden sind, eine hydrophobe Gruppe oder ein Wasserstoffatom;
die Gruppen X und X', die gleich oder verschieden sind, eine Gruppe, die eine Aminofunktion trägt, die gegebenenfalls eine hydrophobe Gruppe aufweist, oder die Gruppe L";
die Gruppen L, L' und L", die gleich oder verschieden sind, eine von einem Diisocyanat abgeleitete Gruppe;
die Gruppen P und P', die gleich oder verschieden sind, eine Gruppe, die eine Aminofunktion aufweist, die gegebenenfalls eine hydrophobe Gruppe trägt;
Y eine hydrophile Gruppe;
r eine ganze Zahl im Bereich von 1 bis 100 und vorzugsweise 1 bis 50, insbesondere 1 bis 25;
n, m und p jeweils unabhängig voneinander Werte im Bereich von 0 bis 1 000;
wobei das Molekül mindestens eine protonierte Aminofunktion oder quaternisierte Aminofunktion und mindestens eine hydrophobe Gruppe aufweist;
- (II) quaternisierten Cellulosederivaten und Polyacrylaten mit nichtcyclischen aminierten Seitengruppen.

24. Zusammensetzung nach Anspruch 1, **dadurch gekennzeichnet, dass** das oder die amphoteren assoziativen Polymere enthalten oder hergestellt werden, in dem copolymerisiert werden:
1) mindestens ein Monomer der Formel (Ia) oder (Ib): 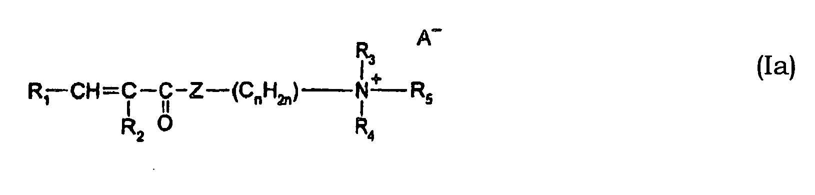 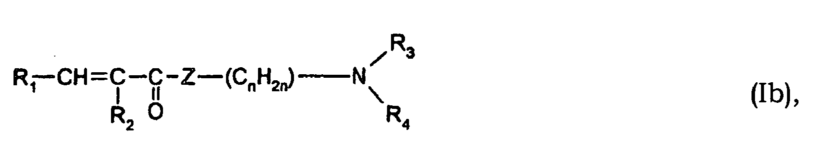 worin die Gruppen R₁ und R₂, die gleich oder verschieden sind, ein Wasserstoffatom oder Methyl bedeuten, die Gruppen R₃, R₄ und R₅, die gleich oder verschieden sind, eine geradkettige oder verzweigte Alkylgruppe mit 1 bis 30 Kohlenstoffatomen bedeuten,
Z eine Gruppe NH oder ein Sauerstoffatom bedeutet,
n eine ganze Zahl von 2 bis 5 ist,
A⁻ ein Anion ist, das von einer organischen oder anorganischen Säure stammt, beispielsweise ein Methosulfatanion oder ein Halogenid, wie Chlorid oder Bromid;
2) mindestens ein Monomer der Formel (II)
R₆-CH = CR₇ COOH (II)
worin die Gruppen R₆ und R₇, die gleich oder verschieden sind, ein Wasserstoffatom oder die Methylgruppe bedeuten; und
3) mindestens ein Monomer der Formel (III) :
R₆-CH = CR₇- COXR₈ (III),
worin die Gruppen R₆ und R₇, die gleich oder verschieden sind, ein Wasserstoffatom oder die Methylgruppe bedeuten, X ein Sauerstoffatom oder ein Stickstoffatom ist und die Gruppe R₈ eine geradkettige oder verzweigte Alkylgruppe mit 1 bis 30 Kohlenstoffatomen bedeutet;
wobei mindestens ein Monomer der Formel (Ia), (Ib) oder (III) mindestens eine Fettkette aufweist.

25. Zusammensetzung nach einem der Ansprüche 17 bis 24, **dadurch gekennzeichnet, dass** das oder die assoziativen Polymere in einem Mengenanteil von 0,1 bis 10 Gew.-%, bezogen auf das Gesamtgewicht der Zusammensetzung, enthalten sind.

26. Zusammensetzung nach Anspruch 25, **dadurch gekennzeichnet, dass** das oder die assoziativen Polymere in einem Mengenanteil von 0,5 bis 5 Gew.-%, bezogen auf das Gesamtgewicht der Zusammensetzung, enthalten sind.

27. Zusammensetzung nach Anspruch 26, **dadurch gekennzeichnet, dass** das oder die assoziativen Polymere in einem Mengenanteil von 1 bis 3 Gew.-%, bezogen auf das Gesamtgewicht der Zusammensetzung, enthalten sind.

28. Zusammensetzung nach einem der vorhergehenden Ansprüche, **dadurch gekennzeichnet, dass** sie ferner mindestens ein kationisches oder amphoteres Polymer enthält, das von den assoziativen Polymeren und den Polymeren mit Einheiten (i) Acrylamid, (ii) Dialkyldiallylammoniumhalogenid und (iii) Vinylcarbonsäure nach einem der vorhergehenden Ansprüche verschieden ist.

29. Zusammensetzung nach Anspruch 28, **dadurch gekennzeichnet, dass** das kationische oder amphotere Polymer ausgewählt ist unter:
-1/ Homopolymeren von Dimethyldiallylammoniumchlorid;
-2/ Polymeren mit Wiederholungseinheiten der folgenden Formeln (W) oder (U): 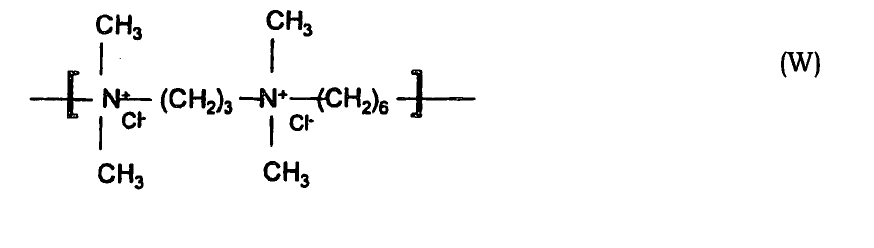 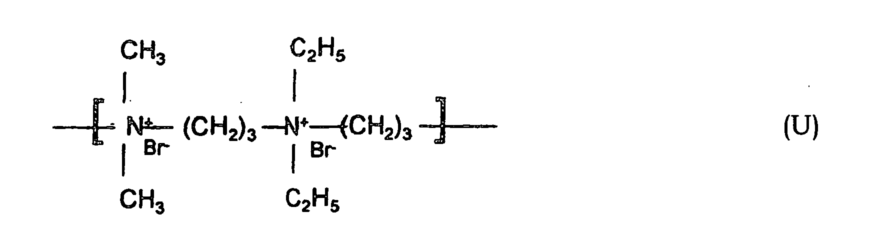
-3/ Polymeren mit Einheiten der folgenden Formel (IX): 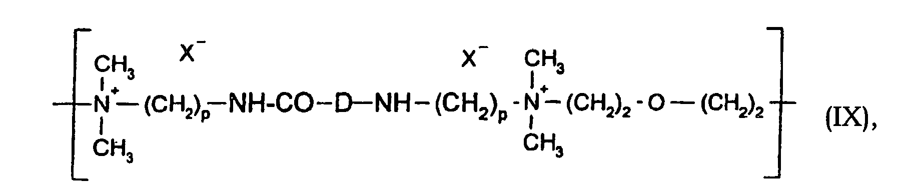 worin p 3 ist und
a) D den Wert Null hat, X ein Chloratom bedeutet,
b) D eine Gruppe -(CH₂)₄-CO- bedeutet, X ein Chloratom ist,
c) D die Gruppe -(CH₂)₇-CO- bedeutet, X ein Chloratom ist,
d) ein "Blockcopolymer", das aus Einheiten gebildet ist, die den unter a) und b) oben beschriebenen Polymeren entsprechen;
-4/ Copolymeren von Acrylsäure und Dimethyldiallylammoniumchlorid.

30. Zusammensetzung nach Anspruch 28 oder 29, **dadurch gekennzeichnet, dass** das oder die kationischen oder amphoteren Polymere in einem Mengenanteil von 0,01 bis 10 Gew.-%, bezogen auf das Gesamtgewicht der Zusammensetzung, enthalten sind.

31. Zusammensetzung nach einem der vorhergehenden Ansprüche, **dadurch gekennzeichnet, dass** sie mindestens einen grenzflächenaktiven Stoff, der unter den anionischen, kationischen, nichtionischen oder amphoteren oder zwitterionischen grenzflächenaktiven Stoffen ausgewählt ist, in einem Mengenanteil von mindestens 0,01 Gew.-%, bezogen auf das Gesamtgewicht der Zusammensetzung, enthält.

32. Zusammensetzung nach Anspruch 31, **dadurch gekennzeichnet, dass** der grenzflächenaktive Stoff nichtionisch ist.

33. Zusammensetzung nach Anspruch 7, **dadurch gekennzeichnet, dass** sie einen pH-Wert von 3 bis 12 aufweist.

34. Zusammensetzung nach Anspruch 33, **dadurch gekennzeichnet, dass** der pH-Wert im Bereich von 8,5 bis 11 liegt.

35. Zusammensetzung nach Anspruch 7, **dadurch gekennzeichnet, dass** das Oxidationsmittel unter Wasserstoffperoxid, Harnstoffperoxid, Alkalimetallbromaten, Alkalimetallferricyaniden, Salzen von Persäuren, Redoxenzymen gegebenenfalls mit ihrem jeweiligen Donor oder Cofaktor ausgewählt ist.

36. Zusammensetzung nach Anspruch 35, **dadurch gekennzeichnet, dass** es sich bei dem Oxidationsmittel um Wasserstoffperoxid handelt.

37. Zusammensetzung nach Anspruch 35, **dadurch gekennzeichnet, dass** das Oxidationsmittel eine Wasserstoffperoxidlösung mit einem Titer von 1 bis 40 Volumina ist.

38. Verfahren zum Färben von Keratinfasern und insbesondere menschlichen Keratinfasern, besonders Haaren, **dadurch gekennzeichnet, dass** es darin besteht, auf die Fasern mindestens eine Farbmittelzusammensetzung aufzubringen, die in einem zum Färben geeigneten Medium mindestens ein Farbmittel (Direktfarbstoff oder Oxidationsfarbstoff) enthält, wobei die Farbe bei einem alkalischen, neutralen oder sauren pH-Wert mit einer oxidierenden Zusammensetzung gebildet wird, die mindestens ein Oxidationsmittel enthält und die bei der Anwendung mit der Farbmittelzusammensetzung vermischt wird oder die getrennt davon anschließend ohne zwischenzeitliches Spülen aufgebracht wird, wobei mindestens ein nichtionisches, kationisches oder amphoteres assoziatives Polymer und mindestens ein Polymer mit den Einheiten (i) Acrylamid, (ii) Dialkyldiallylammoniumhalogenid und (iii) Vinylcarbonsäure, wie es in den Ansprüchen 9 bis 17 definiert ist, in der Farbmittelzusammensetzung oder in der oxidierenden Zusammensetzung oder in beiden Zusammensetzungen enthalten sind.

39. Verfahren nach Anspruch 38, **dadurch gekennzeichnet, dass** das oder die assoziativen Polymere und das oder die Polymere mit den Einheiten (i) Acrylamid, (ii) Dialkyldiallylammoniumhalogenid und (iii) Vinylcarbonsäure in der Farbmittelzusammensetzung enthalten sind.

40. Verfahren nach den Ansprüchen 38 oder 39, **dadurch gekennzeichnet, dass** es darin besteht, auf die trockenen oder feuchten Keratinfasern die gebrauchsfertige Zusammensetzung aufzutragen, die bedarfsgemäß bei der Anwendung aus der Farbmittelzusammensetzung und der oxidierenden Zusammensetzung hergestellt wird, sie während einer Einwirkzeit von etwa 1 bis 60 Minuten und vorzugsweise 5 bis 45 Minuten einwirken zu lassen, die Fasern zu spülen, gegebenenfalls mit Haarwaschmittel zu waschen, nochmals zu spülen und zu trocknen.

41. Vorrichtung mit mehreren Abteilungen oder "Kit" zum Färben von Keratinfasern und insbesondere menschlichen Keratinfasern, besonders Haaren, **dadurch gekennzeichnet, dass** sie mindestens zwei Abteilungen aufweist, wobei eine Abteilung eine Farbmittelzusammensetzung, die in einem zum Färben geeigneten Medium mindestens ein Farbmittel (Direktfarbstoff oder Oxidationsfarbstoff) enthält, und eine weitere Abteilung eine oxidierende Zusammensetzung enthält, die in einem zum Färben geeigneten Medium ein Oxidationsmittel enthält, wobei mindestens ein nichtionisches, kationisches oder amphoteres Polymer und mindestens ein Polymer mit den Einheiten (i) Acrylamid, (ii) Dialkyldiallylammoniumhalogenid und (iii) Vinylcarbonsäure, das in den Ansprüchen 9 bis 17 definiert ist, in der Farbmittelzusammensetzung oder in der oxidierenden Zusammensetzung oder in beiden Zusammensetzungen enthalten sind.
